# EUROPÄISCHE PATENTANMELDUNG

(11) **EP 4 137 194 A1**
(43) Veröffentlichungstag der Anmeldung: **22.02.2023**
(21) Anmeldenummer: 21192477.4
(22) Anmeldetag: 20.08.2021
(51) Int. Cl.: A61M 60/178, A61M 60/422, F04D 29/048

(54) **PUMPE**

(71) Anmelder: Berlin Heart GmbH, 12247 Berlin (DE)
(72) Erfinder: Dr.-Ing. Richert, Hendryk, 12487 Berlin (DE); Peters, Oliver, 14129 Berlin (DE)
(74) Vertreter: Pfenning, Meinig & Partner mbB

(57) **Zusammenfassung**

Pumpe, insbesondere eine Blutpumpe, umfassend eine Pumpenkammer mit einer Wandung und einer Fluidverbindung zwischen einem Einlass und einem Auslass; einen Impeller; einen Motorstator; eine an der Pumpenkammer angeordnete Steuerspule, um zusammen mit ersten Rotormagneten eine steuerbare Axialkraft auf den Rotor auszuüben; eine Steuereinheit zum Bereitstellen eines Steuerstroms für die Steuerspule, um den Rotor in einer von der Wandung der Pumpenkammer axial beabstandeten Position zu halten; einen ersten Kammermagneten nahe der Wicklung der Steuerspule und dem ersten Rotormagneten gegenüberliegend angeordnet, um zusammen mit dem ersten Rotormagneten eine Kraft zwischen dem ersten Rotormagneten und dem ersten Kammermagneten bereitzustellen, um den Rotor relativ zur Wandung der Pumpenkammer auszurichten.

## Beschreibung

Die Anmeldung betrifft eine Pumpe, insbesondere eine Pumpe mit einem magnetisch gelagerten Rotor und hierbei bevorzugt eine Blutpumpe mit einem magnetisch gelagerten Rotor, ein Verfahren zum Betreiben der Pumpe sowie eine Verwendung der Pumpe. Die Pumpe kann beispielsweise in einem Herzunterstützungssystem verwendet werden.

Herzunterstützungssysteme werden eingesetzt, um einen Blutfluss im Körper aufrechtzuerhalten, insbesondere in Fällen, in denen ein Herz dies nicht in ausreichendem Maße gewährleisten kann.

Blutpumpen mit einem magnetisch gelagerten Rotor, kurz magnetisch gelagerte Blutpumpen, werden gegenwärtig als Stand der Technik im Bereich der Blutpumpen, die beispielsweise in Herzunterstützungssystemen einsetzbar sind, betrachtet. Dabei kann jedoch die Komplexität eines Aufbaus mit Magnetlagern einer Miniaturisierung der Blutpumpe entgegenstehen.

Der vorliegenden Erfindung liegt die Aufgabe zugrunde, eine weitere Miniaturisierung einer Pumpe und insbesondere einer magnetisch gelagerten Blutpumpe bei gleichzeitiger Erhaltung wichtiger funktionaler Eigenschaften wie Leistung, Steuerbarkeit, Sicherheit und Hämokompatibilität zu ermöglichen.

Die Aufgabe wird gemäß den Merkmalen der unabhängigen Ansprüche gelöst. Weitere vorteilhafte Ausführungsformen der Erfindung sind in den auf die unabhängigen Ansprüche rückbezogenen Ansprüchen angegeben.

Offenbart wird eine Pumpe umfassend eine Pumpenkammer mit einer Wandung und einer Fluidverbindung für einen Fluidfluss zwischen einem Einlass und einem Auslass der Pumpenkammer. Bei der Pumpe kann es sich beispielsweise, aber auch insbesondere um eine Blutpumpe handeln und der Fluidfluss kann beispielsweise, aber auch insbesondere ein Blutfluss sein. Der Fluidfluss kommt zustande, indem das Fluid durch den Einlass, der eine Öffnung der Pumpenkammer ist, in die Pumpenkammer strömt, die Pumpenkammer durchläuft und die Pumpenkammer durch den Auslass, der eine Öffnung der Pumpenkammer ist, verlässt. Die Wandung begrenzt die Pumpenkammer, die als ein Hohlraum ausgebildet ist. Die Pumpe hat die Aufgabe, den Fluidfluss zu treiben.

Die Pumpe umfasst ferner einen in der Pumpenkammer angeordneten, mit einem Rotor verbundenen Impeller, ausgebildet zum Drehen um eine Rotationsachse, die am Einlass ausgerichtet ist und eine axiale Richtung definiert. Der Impeller ist ausgebildet, den Fluidfluss vom Einlass zum Auslass zu treiben. Der Impeller kann beispielsweise mit dem Rotor starr verbunden sein. Die Ausrichtung des Rotors und/oder des Impellers kann beispielsweise so erfolgen, dass die Rotationsachse in etwa durch eine Mitte des Einlasses verläuft. Es wird angenommen, dass der Rotor nur geringe translatorische Bewegungen ausführt, so dass, mit dem Ziel einer einfacheren und verständlicheren Beschreibung, die Rotationsachse hierein bezüglich der Pumpenkammer als feststehend angesehen werden kann. Der Impeller kann einen oder mehrere Abschnitte aufweisen, beispielsweise Flügel und/oder Schaufeln, mit denen das Fluid, beispielsweise Blut, bewegt werden kann, mit denen der Fluidfluss antreibbar ist. Beispielsweise kann der Impeller eine im Wesentlichen scheibenförmige Gestalt aufweisen, wobei bei einer solchen Aussage insbesondere auch eine eng anliegende gedachte Einhüllende um den Impeller betrachtet wird, in die beispielsweise die Flügel oder Schaufeln integriert sein können. Scheibenförmig ist beispielsweise eine im Wesentlichen zylindrische Form, wobei die Zylinderhöhe geringer als der Durchmesser der Zylindergrundfläche ist, beispielsweise um einen Faktor 2 bis 3.

Außerdem umfasst die Pumpe einen an der Pumpenkammer angeordneten Motorstator, wobei der Motorstator mindestens eine Motorspule umfasst, die ausgebildet ist, ein Motormagnetfeld bereitzustellen, das ausgebildet ist, mit einem Magnetfeld eines am Impeller angeordneten Motormagneten zusammenzuwirken, um den Impeller rotatorisch anzutreiben. Es ist dabei möglich, dass der Motorstator mehrere Motorspulen umfasst. Diese können beispielsweise ringförmig angeordnet sein. Wicklungen der Spulen können beispielsweise jeweils Windungen um die axiale Richtung herum aufweisen. Pumpenkammer und Motorstator können durch die Wandung voneinander getrennt sein.

Es ist darüber hinaus möglich, dass der Motormagnet mehrere Motormagneten umfasst. Die Motormagneten können beispielsweise ringförmig am Impeller angeordnet sein. Dabei ist es auch denkbar, dass die Motormagneten den Motorspulen gegenüberliegen. Die Magnetfeldrichtung des oder der Motormagneten kann in eine axiale Richtung weisen. Bei benachbarten Motormagneten besteht außerdem die Möglichkeit, dass die Magnetfeldrichtung um 180° voneinander abweicht, wobei auch andere Abweichungen möglich sind, wie beispielsweise 90°. Motorstator und Motormagneten können beispielsweise nach einem Prinzip eines Axialflussmotors zusammenzuwirken.

Außerdem weist die Pumpe eine an der Pumpenkammer angeordnete Steuerspule auf, wobei eine oder mehrere Windungen einer Wicklung der Steuerspule an der Wandung um die Pumpenkammer geführt sind und die Steuerspule ausgebildet ist, mit einem Steuerstrom ein Magnetfeld zu erzeugen, das ausgebildet ist, mit einem am Rotor angeordneten ersten Rotormagneten eine steuerbare Axialkraft auf den Rotor in der axialen Richtung auszuüben.

Es besteht die Möglichkeit, dass die Steuerspule in Einlassnähe angeordnet ist. Beispielsweise ist ihre Anordnung so möglich, dass ihre Wicklung, beispielsweise vollständig, in axialer Richtung betrachtet, zwischen Einlass und Rotor angeordnet ist. Es besteht andererseits auch die Möglichkeit einer anderen Anordnung, beispielsweise so, dass sich die Steuerspule in der Nähe des Impellers befindet. Dabei ist es jeweils denkbar, dass sich die Steuerspule nicht in der Pumpenkammer befindet, sondern, ausgehend von der Pumpenkammer hinter der Wandung positioniert ist. Die Steuerspule kann auch in die Wandung integriert sein. Die eine oder mehreren Windungen der Wicklung der Spule können um eine axiale Richtung herum geführt sein. Es ist auch möglich, dass die steuerbare Axialkraft in Richtung Einlass oder vom Einlass weg wirkt.

Beispielsweise kann der erste Rotormagnet permanentmagnetisch und ringförmig gestaltet sein und dabei an einem Umfang des Rotors, beispielsweise an einem Innenumfang und/oder einem Außenumfang, um den Rotor herum geführt sein, beispielsweise so, dass der erste Rotormagnet entlang des Umfangs einen in der radialen Richtung im Wesentlichen konstanten Abstand zur Rotationsachse aufweist. Der erste Rotormagnet kann dann beispielsweise eine Magnetisierung und somit eine Magnetfeldrichtung in der radialen Richtung aufweisen. Magnetisierung umfasst unter anderem die Anordnung von Nord- und Südpol eines Magneten zueinander. Eine Magnetisierungsrichtung gibt eine vom Nord- zum Südpol des Magneten geradlinig verlaufende Richtung an. Der Begriff Magnetisierung kann im Folgenden insbesondere bei Richtungsangaben auch im Sinne einer Magnetisierungsrichtung verwendet werden.

Der erste Rotormagnet kann aber auch eine Magnetisierung in axialer Richtung aufweisen und er kann auch in ein oder mehrere Abschnitte, beispielsweise in der axialen Richtung oder auch entlang seines Umfangs, mit jeweils eigener Magnetisierung unterteilt sein. Die Magnetisierungsrichtung der verschiedenen Abschnitte kann voneinander abweichen, beispielsweise auch entgegengesetzt sein. Es ist beispielsweise auch eine Anordnung in der Form eines Halbach-Arrays denkbar, wobei in einem solchen Fall der Rotormagnet in Abschnitte unterteilt sein kann, deren Magnetisierungsrichtung jeweils Vorgaben für ein Halbach-Array entspricht. Der Rotormagnet kann beispielsweise als Neodymmagnet ausgebildet sein, aber auch andere Magnet-Materialen sind vorstellbar.

Die Pumpe weist darüber hinaus eine Steuereinheit auf, dazu ausgebildet, den Steuerstrom für die Steuerspule bereitzustellen, um den Rotor in einer von der Wandung der Pumpenkammer axial beabstandeten Position zu halten. Bei der Steuereinheit kann es sich, beispielsweise, um einen Mikrocontroller, einen Digitalen Signalprozessor, ein FPGA (field programmable gate array) oder einen ASIC (application specific integrated circuit) oder auch eine Kombination davon handeln. Darüber hinaus können weitere elektronische Komponenten und entsprechende Schaltungen zur Steuereinheit hinzukommen, beispielsweise ein oder mehrere Sensoren, wie zum Beispiel ein Abstands- und/oder Positionssensor, ein Magnetfeldsensor, ein Drehzahlsensor, ein Drucksensor, ein Sensor zur Spannungsmessung und/oder Strommessung, ein Flusssensor für das Fluid und dergleichen, aber auch beispielsweise ein Verstärker, ein Filter, ein Umrichter oder auch eine Schaltung, die der Kommunikation dient, eine Spannungsversorgung usw.

Die Pumpe weist auch einen ersten Kammermagneten auf, der an der Pumpenkammer, nahe der Wicklung der Steuerspule und dem ersten Rotormagneten gegenüberliegend angeordnet und ausgebildet ist, zusammen mit dem ersten Rotormagneten eine Kraft zwischen dem ersten Rotormagneten und dem ersten Kammermagneten bereitzustellen, um den Rotor relativ zur Wandung der Pumpenkammer auszurichten. Nahe der Wicklung bedeutet hier, dass der erste Kammermagnet beispielsweise angrenzend an die Wicklung der Steuerspule positioniert sein kann oder nur in einem geringen Abstand, bei dem das Magnetfeld des dem Kammermagneten gegenüberliegend angeordneten ersten Rotormagneten mit dem Magnetfeld der Steuerspule wechselwirken kann.

Der erste Kammermagnet kann beispielsweise eine ringförmige Gestalt aufweisen. Dabei ist es denkbar, dass der erste Kammermagnet in die Wandung der Pumpenkammer, beispielsweise auch in einen Vorsprung der Wandung der Pumpenkammer, integriert ist oder auch, von der Pumpenkammer ausgehend, hinter der oder auch vor der Wandung positioniert ist. Der erste Kammermagnet kann beispielsweise entlang eines Umfangs einen im Wesentlichen konstanten Abstand zur Rotationsachse aufweisen. Der erste Kammermagnet kann sich auch ringförmig um die Rotationsachse erstrecken. Es besteht ferner die Möglichkeit, dass der erste Kammermagnet beispielsweise eine Magnetisierung in der radialen Richtung aufweist. Er kann aber auch eine Magnetisierung in der axialen Richtung besitzen und er kann auch in ein oder mehrere Abschnitte, beispielsweise in der axialen Richtung oder auch entlang seines Umfangs, mit jeweils eigener Magnetisierung unterteilt sein. Die Magnetisierungsrichtung der verschiedenen Abschnitte kann voneinander abweichen, beispielsweise auch entgegengesetzt sein. Es ist aber beispielsweise auch eine Anordnung in der Form eines Halbach-Arrays denkbar, wobei in einem solchen Fall der erste Kammermagnet in Abschnitte unterteilt sein kann, deren Magnetisierungsrichtung jeweils den Vorgaben für ein Halbach-Array entspricht. Der erste Kammermagnet kann beispielsweise als Neodymmagnet ausgebildet sein, aber auch andere Magnet-Materialen sind vorstellbar.

Die Pumpe kann auch ein zweites Magnetlager aufweisen, wobei das zweite Magnetlager beispielsweise einen zweiten Kammermagneten umfassen kann, der an der Pumpenkammer dort angeordnet ist, wo die Rotationsachse auf die Wandung trifft. Das zweite Magnetlager kann darüber hinaus einen dem zweiten Kammermagneten gegenüberliegend am Rotor angeordneten zweiten Rotormagneten aufweisen. Es besteht die Möglichkeit, dass das zweite Magnetlager eine Abstoßungskraft zwischen dem zweiten Rotormagneten und dem zweiten Kammermagneten bereitstellt, um den Rotor oder einen Abschnitt des Rotors in einer von der Wandung der Pumpenkammer in einer radialen Richtung, die senkrecht zu der axialen Richtung definiert ist, beabstandeten Position zu halten. Das zweite Magnetlager kann beispielsweise nach einem Prinzip eines Permanentmagnetlagers bzw. eines passiven Magnetlagers funktionieren.

Der zweite Kammermagnet kann beispielsweise eine zylindrische oder auch eine ringförmige Gestalt aufweisen. Die Anordnung des zweiten Kammermagneten erfolgt an der Pumpenkammer. Dabei ist es denkbar, dass der zweite Kammermagnet in die Wandung der Pumpenkammer, beispielsweise auch in oder an einen Vorsprung der Wandung der Pumpenkammer, integriert ist oder auch, von der Pumpenkammer ausgehend, hinter der oder auch vor der Wandung positioniert ist. Der zweite Kammermagnet kann beispielsweise entlang eines Umfangs einen im Wesentlichen konstanten Abstand zur Rotationsachse aufweisen. Die Rotationsachse kann auch durch den zweiten Kammermagneten hindurchverlaufen und der zweite Kammermagnet kann sich auch ringförmig um die Rotationsachse erstrecken. Es besteht ferner die Möglichkeit, dass der zweite Kammermagnet beispielsweise eine Magnetisierung in der radialen Richtung aufweist. Er kann aber auch eine Magnetisierung in axialer Richtung besitzen und er kann auch in ein oder mehrere Abschnitte, beispielsweis in der axialen Richtung oder auch entlang seines Umfangs, mit jeweils eigener Magnetisierung unterteilt sein. Die Magnetisierungsrichtung der verschiedenen Abschnitte kann voneinander abweichen, beispielsweise auch entgegengesetzt sein. Es ist aber beispielsweise auch eine Anordnung in der Form eines Halbach-Arrays denkbar, wobei in einem solchen Fall der zweite Kammermagnet in Abschnitte unterteilt sein kann, deren Magnetisierungsrichtung jeweils den Vorgaben für ein Halbach-Array entspricht. Der zweite Kammermagnet kann beispielsweise als Neodymmagnet ausgebildet sein, aber auch andere Magnet-Materialen sind vorstellbar.

Es ist denkbar, dass der zweite Rotormagnet permanentmagnetisch und ringförmig gestaltet ist und dabei an einem Umfang des Rotors, beispielsweise an einem Innenumfang und/oder einem Außenumfang, um den Rotor herum geführt ist, beispielsweise so, dass der zweite Rotormagnet entlang des Umfangs einen in der radialen Richtung im Wesentlichen konstanten Abstand zur Rotationsachse aufweist. Der zweite Rotormagnet kann dann beispielsweise eine Magnetisierung in der radialen Richtung aufweisen. Er kann aber auch eine Magnetisierung in axialer Richtung aufweisen und er kann auch in ein oder mehrere Abschnitte, beispielsweise in der axialen Richtung oder auch entlang seines Umfangs, mit jeweils eigener Magnetisierung unterteilt sein. Die Magnetisierungsrichtung der verschiedenen Abschnitte kann voneinander abweichen, beispielsweise auch entgegengesetzt sein. Es ist beispielsweise auch eine Anordnung in der Form eines Halbach-Arrays denkbar, wobei in einem solchen Fall der Rotormagnet in Abschnitte unterteilt sein kann, deren Magnetisierungsrichtung jeweils Vorgaben für ein Halbach-Array entspricht. Der Rotormagnet kann beispielsweise als Neodymmagnet ausgebildet sein, aber auch andere Magnet-Materialen sind vorstellbar.

In einigen Ausführungsformen besteht die Möglichkeit, dass der erste Rotormagnet und der zweite Rotormagnet identisch sind. Dies kann beispielsweise vorteilhaft sein, wenn es darauf ankommt, eine Anzahl notwendiger Bauteile in der Herstellung der Pumpe zu verringern und/oder die Anzahl der Fertigungsschritte zu reduzieren. Diese Möglichkeit bietet sich beispielsweise dann an, wenn die Magnetisierung von erstem und zweitem Rotormagnet identisch sein soll und beide aneinander angrenzend anzuordnen sind.

In einigen Ausführungsformen ist es denkbar, dass der erste Rotormagnet und der zweite Rotormagnet voneinander verschieden sind. Dies kann beispielsweise vorteilhaft sein, wenn es darauf ankommt, die vom jeweiligen Rotormagneten, beispielsweise im Zusammenwirken mit dem jeweils zugehörigen Kammermagneten, wie oben beschrieben, oder beispielsweise der Steuerspule, bereitgestellten Kräfte auf den Rotor separat einzustellen, weil dies beispielsweise einfacher sein kann, oder beispielsweise den jeweiligen Kraftangriffspunkt auf den Rotor separat und somit beispielsweise besser zu justieren.

In einem Ausführungsbeispiel ist es möglich, dass der Rotor um die Rotationsachse eine Ausnehmung aufweist, und dazu ausgebildet ist, den Fluidfluss durch die Ausnehmung zu führen. Der Rotor kann zum Beispiel in einem Abschnitt als Hohlrohr oder auch als Bohrung gestaltet sein. Es besteht die Möglichkeit, dass der Fluidfluss, beispielsweise der Blutfluss, durch die Ausnehmung strömen kann. Damit kann in einem Beispiel auch ein Abstand zwischen dem ersten Rotormagneten und dem ersten Kammermagneten gering gestaltet werden, ohne dabei den Fluidfluss durch die Pumpe ebenfalls gering gestalten zu müssen. Da die Kraftwirkung zwischen dem ersten Rotormagneten und dem ersten Kammermagneten mit kleinerem Abstand zueinander größer wird, können die Magneten bei geringerem Abstand kleiner und/oder weniger stark ausgelegt sein. Dies ermöglicht beispielsweise, bei gleichbleibenden Leistungsparametern ein Volumen der Pumpe zu reduzieren und/oder Herstellungskosten zu senken.

Es ist denkbar, dass die Kraft zwischen dem ersten Rotormagneten und dem ersten Kammermagneten eine Anziehungskraft ist und der erste Rotormagnet und der erste Kammermagnet ein erstes Magnetlager bilden, das ausgebildet ist, den Rotor in einer Kipprichtung, die eine Drehachse in der radialen Richtung aufweist, auszurichten. So kann man sich beispielsweise vorstellen, dass der erste Rotormagnet und der erste Kammermagnet in der Nähe eines Außenumfangs des Impellers angeordnet sind. So kann die Anziehungskraft zwischen erstem Rotormagneten und erstem Kammermagneten dafür sorgen, dass der Impeller an seinem Außenumfang immer in der der Nähe des Kammermagneten positioniert ist, also keine Drehung in der Kipprichtung, die auch als Kippung bezeichnet werden kann, erfährt. Der Impeller kann so in einer für das Antreiben des Fluidflusses günstigen Position gehalten werden.

In einem Ausführungsbeispiel ist es ferner möglich, dass die Kraft zwischen dem ersten Rotormagneten und dem ersten Kammermagneten eine Abstoßungskraft in einer radialen Richtung ist, die senkrecht zu der axialen Richtung definiert ist, und der erste Rotormagnet und der erste Kammermagnet ein erstes Magnetlager bilden, das ausgebildet ist, den Rotor in einer von der Wandung der Pumpenkammer in der radialen Richtung beabstandeten Position zu halten. Das erste Magnetlager kann beispielsweise nach einem Prinzip eines Permanentmagnetlagers bzw. eines passiven Magnetlagers funktionieren. Das erste Magnetlager kann in diesem Fall ein in der radialen Richtung stabiles Magnetlager sein. Es ist auf diese Weise beispielsweise auch möglich, den Rotor, in Zusammenwirken mit dem zweiten Magnetlager, vollständig in der radialen Richtung zu stützen. Wenn erstes und zweites Magnetlager voneinander beabstandet angeordnet sind, beispielsweise in der axialen Richtung, kann auch eine Bewegung des Rotors in der Kipprichtung unterdrückt oder zumindest vermindert werden. Dies kann beispielsweise so realisiert werden, in dem, bezüglich der axialen Richtung, das erste Magnetlager in Einlassnähe angeordnet wird und das zweite Magnetlager in Auslassnähe, beispielsweise auch in der Nähe des Impellers, angeordnet wird. In einem solchen Fall kann der Rotor, in der axialen Richtung betrachtet, einen ersten Abschnitt nahe dem Einlass und einen zweiten Abschnitt entfernt vom Einlass aufweisen. In einem Beispiel ist es denkbar, dass das erste Magnetlager im ersten Abschnitt ist und das zweite Magnetlager im zweiten Abschnitt angeordnet ist. Der Impeller kann dann beispielsweise auch im zweiten Abschnitt angeordnet sein. Erster und zweiter Abschnitt können fließend ineinander übergehen.

Es ist im weiteren denkbar, dass die Steuerspule ein zwischen der Wicklung und der Fluidverbindung und/oder auf einer dem Rotor abgewandten Seite der Steuerspule angeordnetes magnetisch leitfähiges Material aufweist. Ein magnetisch leitfähiges Material kann beispielsweise eine hohe relative magnetische Permeabilität aufweisen, wie zum Beispiel ein ferromagnetischer Stoff, beispielsweise Eisen. Typische Werte für die relative magnetische Permeabilität ferromagnetischer Stoffe können beispielsweise im Bereich zwischen 300 und 10000 (ohne Einheit) liegen, wobei für bestimmte Stoffe, wie beispielsweise Mumetalle, amorphe Metalle oder auch nanokristalline Metalle auch Werte deutlich darüber bekannt sind, beispielsweise bis 150000 oder sogar bis 500000. Bei bestimmten Stoffen sind auch geringere relative magnetische Permeabilitäten bekannt, die beispielsweise bei Ferriten bis etwa auf 4 absinken können. Das magnetisch leitfähige Material kann dazu dienen, die Leitung eines von der Steuerspule bereitgestellten magnetischen Flusses zu verbessern und den magnetischen Fluss zu führen, so dass das von der Steuerspule erzeugte Magnetfeld beim Zusammenwirken mit dem Magnetfeld des ersten Rotormagneten eine größere axiale Kraft erzeugen kann. Die Führung des magnetischen Flusses erfolgt dabei so, dass magnetisch leitfähiges Material dort bereitgestellt sein kann, wo keine Kraftwirkung zwischen dem Magnetfeld der Steuerspule und dem Magnetfeld des ersten Rotormagneten erfolgen soll. Dementsprechend kann das magnetisch leitfähige Material auf einer dem Rotor abgewandten Seite der Steuerspule angeordnet sein, also beispielsweise auf einer radial von der Rotationsachse entfernten Seite der Steuerspule oder auch auf einer axial dem Einlass zugewandten Seite der Steuerspule. Darüber hinaus ist die Anordnung des magnetisch leitfähigen Materials auf einer radial der Rotationsachse zugewandten Seite der Steuerspule möglich, wenn ausgehend von dort keine axiale Kraft im Zusammenwirken von Steuerspule und erstem Rotormagneten erzeugt werden soll. Dies kann beispielsweise der Fall sein, wenn die Wicklung der Steuerspule vollständig zwischen dem Einlass und einem dem Einlass zugewandten Ende des Rotors oder auch, in der axialen Richtung betrachtet, zwischen erstem Rotormagneten und Impeller angeordnet ist.

Jedoch sind hier auch andere, alternative und/oder zusätzliche Möglichkeiten einer Anordnung denkbar. Beispielsweise kann die Steuerspule ein magnetisch leitfähiges Material aufweisen, das auf einer der Fluidverbindung abgewandten Seite der Steuerspule und/oder einer dem Rotor zugewandten Seite der Steuerspule angeordnet ist. Die der Fluidverbindung abgewandte Seite der Steuerspule kann auch gleichzeitig die radial von der Rotationsachse entfernte Seite der Steuerspule sein oder mit dieser teilweise übereinstimmen. Es ist darüber hinaus denkbar, dass nicht von allen dem Rotor zugewandten Bereichen der Steuerspule ausgehend eine magnetische Kraftwirkung mit dem ersten Rotormagneten erzeugt werden soll. An diesen Bereichen kann deshalb auch das magnetisch leitfähige Material angeordnet sein. Da wesentliche Anteile des von der Steuerspule erzeugten magnetischen Flusses im magnetisch leitfähigen Material lokalisiert sind, ist ausgehend von den Bereichen, in denen das magnetisch leitfähige Material angeordnet ist, zwischen Steuerspule und erstem Rotormagneten im Wesentlichen keine Kraftwirkung zu erwarten. Um eine Kraftwirkung zwischen Steuerspule und erstem Rotormagneten zu erzielen, kann es vorteilhaft sein, zumindest an einem Bereich der Steuerspule kein magnetisch leitfähiges Material anzuordnen.

Die Vorteile, die sich aus der Anordnung des magnetisch leitfähigen Materials ergeben sind zumindest zweifach: Zum einen kann durch die Anordnung des magnetisch leitfähigen Materials die Kraftwirkung zwischen Steuerspule und erstem Rotormagneten bei gleichbleibendem Steuerstrom erhöht werden. Darüber hinaus kann damit auch ein Ort der Kraftwirkung bestimmt werden. Die Pumpe kann somit eine höhere Effizienz erreichen. Das kann beispielsweise auch bedeuten, dass bei gleichbleibender Leistung die Pumpe kleiner ausgelegt werden kann.

In einer Ausführungsform kann die Pumpe einen an der Steuerspule angeordneten Vorspannmagneten umfassen, der ausgebildet ist, das Magnetfeld der Steuerspule vorzuspannen. Der Vorspannmagnet kann beispielsweise ein Permanentmagnet sein. Vorzuspannen bedeutet in diesem Zusammenhang, dass beispielsweise ein dauerhaftes Magnetfeld vom Vorspannmagneten bereitgestellt wird, dass sich mit dem Magnetfeld der Spule überlagern kann. Dabei ist es beispielsweise möglich, dass der Vorspannmagnet zumindest teilweise an das magnetisch leitfähige Material, das an der Steuerspule angeordnet ist, und/oder an die Steuerspule selbst angrenzt. Der Vorspannmagnet kann auf diese Weise in einen magnetischen Kreis, der die Steuerspule und optional das magnetisch leitfähige Material umfasst, eingebunden sein. Der Vorspannmagnet kann beispielsweise einen magnetischen Fluss in dem magnetischen Kreis bewirken. Der von der Steuerspule erzeugte magnetische Fluss kann sich dann mit dem magnetischen Fluss des Vorspannmagneten im magnetischen Kreis überlagern. Durch die Integration des Vorspannmagneten in den magnetischen Kreis der Steuerspule ist es beispielsweise möglich, auch ohne Steuerstrom eine Kraftwirkung zwischen (dem magnetischen Kreis der) Steuerspule und dem (Magnetfeld des) ersten Rotormagneten zu bewirken. Mit Anlegen eines Steuerstroms an die Steuerspule kann diese Kraftwirkung, je nach Betrag und Vorzeichen des Steuerstroms, verstärkt oder vermindert, also gesteuert werden. Es ist mit dem Vorspannmagneten beispielsweise möglich, einen in der axialen Richtung kraftbezogenen Arbeitspunkt der Pumpe einzustellen, was die Steuerbarkeit der Pumpe verbessert.

Eine andere Möglichkeit, das Magnetfeld der Steuerspule vorzuspannen, besteht beispielsweise darin, eine weitere Spule als Vorspannmagneten zu nutzen oder sogar die Steuerspule selbst, in dem ein Gleichstrom in die Spule bzw. die Steuerspule geleitet wird. Im Gegensatz zu einem Permanentmagnet als Vorspannmagnet kann hierzu jedoch ein elektrischer Strom notwendig sein.

Die Pumpe kann so gestaltet sein, dass der Motormagnet auf einer dem Einlass abgewandten Seite des Impellers angeordnet ist, beispielsweise, wenn auch der Motorstator auf einer dem Einlass abgewandten Seite des Impellers angeordnet ist. Dann kann der Motormagnet so angeordnet sein, dass zwischen Motormagnet und Motorstator ein geringer Abstand besteht. Da mit geringer werdendem Abstand die Kraftwirkung zwischen dem Magnetfeld des Motorstators und dem Magnetfeld des Motormagneten zunimmt, können Motormagnet und Motorstator auf diese Weise bei gleichbleibender Kraftwirkung insgesamt kleiner ausgelegt werden. Damit kann sowohl der zur Verfügung stehende Bauraum effizient genutzt als auch die Steuerbarkeit der Pumpe verbessert werden

Es ist andererseits auch denkbar, dass der Motormagnet auf einer dem Einlass zugewandten Seite des Impellers angeordnet ist. Dies kann beispielsweise dann vorteilhaft sein, wenn beispielsweise auch der Motorstator auf einer dem Einlass zugewandten Seite des Impellers angeordnet ist. Dann kann der Motormagnet so angeordnet sein, dass zwischen Motormagnet und Motorstator ein geringer Abstand besteht. Wie vorstehend erläutert können so Motormagnet und Motorstator auf diese Weise bei gleichbleibender Kraftwirkung insgesamt kleiner ausgelegt werden.

In einem Ausführungsbeispiel besteht die Möglichkeit, dass die Steuereinheit ausgebildet ist, einen Motorspulenstrom so bereitzustellen, dass das Motormagnetfeld zusammen mit dem Magnetfeld des Motormagneten eine Kraft in der axialen Richtung zwischen dem Motormagneten und der Motorspule bewirkt. Beispielsweise kann im Rahmen einer feldorientierten Regelung des Motorspulenstroms ein Strom so berechnet und gesteuert oder geregelt sein, dass nicht nur eine rotatorisch antreibende Kraft über das Magnetfeld des Motorstators und das Magnetfeld der Motormagneten auf den Rotor wirkt, sondern auch eine axial wirkende Kraft auf den Rotor mit dem Motorspulenstrom entsteht. Die axial wirkende Kraft kann beispielsweise so gesteuert sein, dass sie von einer Drehzahl des Rotors abhängig zu- oder abnimmt. Sie kann auch so gesteuert sein, dass sie entsprechend einer Position des Rotors, beispielsweise einer Position in axialer Richtung, zu- oder abnimmt. Die axial wirkende Kraft kann auch so gesteuert sein, dass der Rotor beispielsweise in einer bevorzugten axialen Position gehalten werden kann, beispielsweise in einer axialen Position in einem vorgegebenen Abstand zur Wandung oder auch in einer axialen Position, in der der Leistungsverbrauch der Pumpe gering ist. Letzteres ist beispielsweise für eine axiale Position der Fall, in der sich alle axialen Kräfte auf den Rotor, die nicht auf den Motorspulenstrom und/oder den Steuerstrom der Steuerspule zurückzuführen sind, zu Null ergänzen. Dazu kann beispielsweise ein geeignetes Verfahren in der Steuerung implementiert sein, beispielsweise ein Regelungsverfahren oder ein Steuerungsverfahren oder eine Kombination davon, beispielsweise eine Nullkraftregelung. Das in der Steuerung implementierte Verfahren kann beispielsweise ein von einem der Sensoren bereitgestelltes Signal verarbeiten, beispielsweise ein Positionssignal, ein Magnetsignal, ein Drucksignal, ein Fluidflusssignal, ein Stromsignal, ein Spannungssignal und/oder auch ein Drehzahlsignal.

Sowohl das erste als auch das zweite Magnetlager können jeweils eine radiale Steifigkeit, das heißt eine Steifigkeit in der radialen Richtung, aufweisen, wobei die Steifigkeit ein Verhältnis von einer wirkenden Kraft zu einer damit verbundenen Verschiebungsweglänge angibt. In diesem Falle gibt die radiale Steifigkeit eine am Magnetlager angreifende äußere Kraft in der radialen Richtung an, die erforderlich ist, um den Rotor am Magnetlager um eine vorgegebene Weglänge in der radialen Richtung zu verschieben. Die radiale Steifigkeit ist jeweils eine Eigenschaft des jeweiligen Magnetlagers, die beispielsweise vom jeweiligen Abstand zwischen dem jeweiligen Rotormagneten und dem jeweiligen Kammermagneten und/oder beispielsweise von dem verwendeten Magnet-Material und/oder der jeweiligen Magnetisierung und auch/oder der Magnetisierungsrichtung abhängen kann. Auch die mögliche Anordnung als Halbach-Array kann einen Einfluss auf die jeweilige Steifigkeit des jeweiligen Magnetlagers ausüben. Im Allgemeinen kann die Steifigkeit eines Magnetlagers von der magnetischen Feldstärke zwischen den jeweiligen Magneten abhängen.

In einem Ausführungsbeispiel ist es möglich, dass ein Verhältnis der radialen Steifigkeit des zweiten Magnetlagers zu der radialen Steifigkeit des ersten Magnetlagers zwischen 4:1 und 1,5:1 und bevorzugt zwischen 3:1 und 1,5:1 liegt. Betrachtet werden hier jeweils Beträge der Steifigkeiten. Das erste Magnetlager kann dabei beispielsweise eine geringere Steifigkeit aufweisen als das zweite Magnetlager. So ist es beispielsweise möglich, dass eine am Rotor angreifende Radialkraft, die beispielsweise aus einer Strömung und einer Beschleunigung des Fluids resultieren kann, auf beide Magnetlager (erstes und zweites Magnetlager) derart verteilt wird, dass der Rotor kein oder nur ein geringes Kippmoment erfährt, das zu einer Kippung des Rotors führen kann, und somit eine mögliche Kippung des Rotors unterdrückt oder zumindest vermindert wird. Hierbei spielt eine Rolle, dass ein Produkt aus radialer Kraft (radiale Lagersteifigkeit multipliziert mit einer Auslenkung des Rotors in der radialen Richtung) und Hebelarm zwischen Kraftangriffspunkt und jeweiligen Magnetlagermittelpunkt (in axialer Richtung betrachtet) für beide Magnetlager im Wesentlichen gleich groß ist und somit damit die Auslenkung in der radialen Richtung an beiden Magnetlagern ebenfalls gleich groß ist.

Es ist auch eine Pumpe denkbar, bei der der zweite Rotormagnet und der zweite Kammermagnet so zueinander angeordnet sind, dass eine axiale Kraft in der axialen Richtung vom Einlass weg auf den Rotor wirkt. Beispielsweise können der zweite Rotormagnet und der zweite Kammermagnet in der axialen Richtung zueinander versetzt angeordnet sein.

Es ist darüber hinaus auch möglich, dass ein Quotient aus der radialen Steifigkeit des zweiten Magnetlagers zu der radialen Steifigkeit des ersten Magnetlagers bei einer Rotorverschiebung in der axialen Richtung, in der radialen Richtung oder in der Kipprichtung, die eine Drehachse in der radialen Richtung aufweist, im Wesentlichen konstant bleibt, wobei sich in diesem Beispiel die radiale Steifigkeit des ersten Magnetlagers aus der Steifigkeit von Steuerspule und erstem Kammermagnet mit dem ersten Rotormagneten zusammensetzen kann. Das heißt auch, das Magnetfeld der Steuerspule zusätzlich zum Magnetfeld des ersten Kammermagneten einen Einfluss auf die Steifigkeit des ersten Magnetlagers haben kann.

Mit einer Anordnung des Motors, der den Motorstator mit der Motorspule und den Motormagneten umfasst, und der Steuerspule besteht ferner die Möglichkeit, eine axiale Krafterzeugung so zu gestalten, dass die Summe der vom Motor und der Steuerspule erzeugten Kräfte in der axialen Richtung im Wesentlichen konstant ist.

Es ist hierbei beispielsweise denkbar, dass die Kraft in der axialen Richtung, die vom Motor, der beispielsweise ein Axialflussmotor sein kann, erzeugt wird, größer ist, wenn sich der Rotor, in der axialen Richtung betrachtet, vom Einlass weiter weg befindet, als wenn sich der Rotor, in der axialen Richtung betrachtet, näher am Einlass befindet. Die vom Motor erzeugte Kraft in der axialen Richtung kann dabei eine im Wesentlichen lineare oder annähernd lineare Abhängigkeit von der, in der axialen Richtung betrachteten, Position des Rotors aufweisen. Dabei besteht auch die Möglichkeit, dass die Linearität oder angenäherte Linearität jeweils nur in einem räumlich begrenzten Abschnitt, in dem sich der Rotor aufhält, gilt, wobei es mehrere solche Abschnitte geben kann, in denen beispielsweise auch jeweils ein anderer linearer Zusammenhang gelten kann. Im Allgemeinen ist auch ein nichtlinearer Zusammenhang zwischen axialer Rotorposition und maximaler Kraft auf den Rotor denkbar.

Es ist beispielsweise auch denkbar, dass die Kraft in der axialen Richtung, die von der Steuerspule erzeugt wird, größer ist, wenn sich der Rotor, in der axialen Richtung betrachtet, näher am Einlass befindet, als wenn sich der Rotor, in der axialen Richtung betrachtet, vom Einlass weiter entfernt befindet. Die von der Steuerspule erzeugte Kraft in der axialen Richtung kann dabei eine im Wesentlichen lineare Abhängigkeit vom der, in der axialen Richtung betrachteten, Position des Rotors aufweisen.

Eine Verwendung der Pumpe ist beispielsweise in einem Herzunterstützungssystem möglich, beispielsweise als Blutpumpe oder als magnetgelagerte Blutpumpe.

Es wird auch ein Verfahren zum Betreiben einer Pumpe offenbart. Das Verfahren kann dabei auch das Betreiben einer Blutpumpe oder einer magnetisch gelagerten Blutpumpe betreffen. Beispielsweise kann das Verfahren unter Verwenden einer oben beschriebenen Pumpe durchgeführt werden. Die in Bezug auf die Pumpe beschriebenen Merkmale sind insofern auch auf das Verfahren anwendbar wie auch umgekehrt die Merkmale des Verfahrens auf die Pumpe anwendbar sind.

Das offenbarte Verfahren zum Betreiben einer Pumpe umfasst die Schritte:
- Bereitstellen eines Motormagnetfeldes zum Antreiben eines mit einem Rotor verbundenen Impellers zum Treiben eines Fluidflusses von einem Einlass zu einem Auslass einer Pumpenkammer;
- Bereitstellen, mit einer Steuerspule und einem am Rotor angeordneten ersten Rotormagneten, einer steuerbaren Axialkraft auf den Rotor in Richtung einer Rotationsachse des Rotors, die eine axiale Richtung definiert;
- Bereitstellen eines Steuerstroms für die Steuerspule, um den Rotor in einer von einer Wandung der Pumpenkammer axial beabstandeten Position zu halten, vorzugsweise in einer zeitlich veränderlichen Position, bei der sich die Summe aller Kräfte auf den Rotor außer der steuerbaren Axialkraft in der axialen Richtung zu null addieren;
- Bereitstellen einer Kraft zwischen dem ersten Rotormagneten und einem an der Pumpenkammer nahe einer Wicklung der Steuerspule angeordneten ersten Kammermagneten.

Die genannten Schritte können in dieser Reihenfolge, können aber auch in einer anderen Reihenfolge durchgeführt werden. Einzelne oder auch mehrere der Schritte können auch mehrfach ausgeführt werden.

Im Rahmen des Verfahrens kann eine Nullkraftregelung zum Einsatz kommen, bei der ein dem Betrage nach besonders kleiner Steuerstrom benötigt wird, da der Rotor in der gegebenenfalls zeitlich veränderlichen Position gehalten wird, bei der sich die Summe aller Kräfte auf den Rotor außer der steuerbaren Axialkraft in der axialen Richtung zu null addieren oder im Wesentlichen zu null addieren. Dies ermöglicht einen effizienten Betrieb der Pumpe mit minimierter elektrischer Steuerleistung.

In einem Ausführungsbeispiel kann das Verfahren zum Betreiben einer Pumpe auch einen oder mehrere der folgenden Schritte umfassen:
- Führen des Fluidflusses durch eine Ausnehmung im Rotor um die Rotationsachse des Rotors vom Einlass weg in Richtung Auslass;
- Führen des Fluidflusses, so dass der Fluidfluss am Einlass im Wesentlichen in der axialen Richtung geführt wird und am Auslass in einer Richtung geführt wird, die im Wesentlichen keine axiale Komponente aufweist.
- Führen eines sekundären Fluidflusses zwischen dem ersten Rotormagneten und der Wandung der Pumpenkammer und/oder Führen eines tertiären Fluidflusses zwischen dem Motormagneten und der Wandung der Pumpenkammer.

Das Führen des sekundären und des tertiären Fluidflusses kann beispielsweise dazu dienen, die Pumpenkammer zu spülen. Beispielsweise kann so im Falle einer Blutpumpe das Risiko einer Thrombose verringert werden.

Das Verfahren zum Betreiben einer Pumpe kann auch den folgenden Schritt umfassen: Steuern eines elektrischen Stroms der Steuerspule so, dass ein mittlerer Leistungsverbrauch der Steuerspule minimal wird.

Ebenfalls ist es möglich, dass das Verfahren zum Betreiben einer Pumpe den nachfolgenden Schritt umfasst: Erzeugen einer Abstoßungskraft zwischen einem zweiten Rotormagneten und einem an der Pumpenkammer, dort, wo die Rotationsachse auf eine Wandung der Pumpenkammer trifft, angeordneten zweiten Kammermagneten.

Die Pumpe ist auch in vielen anderen technischen Bereichen als den bisher genannten einsetzbar, in denen ein Fluidfluss getrieben werden muss, wobei es sich bei Fluiden beispielsweise um Gase oder auch um Flüssigkeiten handeln kann. Bevorzugt ist im Rahmen dieser Offenbarung ein Einsatz als magnetisch gelagerte Blutpumpe, beispielsweise in einem Herzunterstützungssystem.

Im Folgenden werden anhand von Figuren Ausführungsbeispiele gezeigt und nachfolgend erläutert. Dabei zeigt:
Fig. 1a: eine schematische Darstellung einer Pumpe;
Fig. 1b: eine schematische Darstellung eines Rotors der Pumpe aus Figur 1a;
Fig. 2a: eine schematische Darstellung einer Pumpe;
Fig. 2b: weitere Details zur Pumpe von Fig. 2a;
Fig. 3a-g: schematische Darstellungen eines ersten Abschnitts des Rotors und einer Steuerspule in einer teilweisen Querschnitts- und teilweisen Perspektivansicht;
Fig. 4: ein Diagramm für eine Möglichkeit zur axialen Krafterzeugung in Abhängigkeit von einer axialen Position des Rotors;
Fig. 5: ein Ablaufdiagramm für ein Verfahren zum Betreiben einer Pumpe.

Wiederkehrende Elemente in den Figuren sind mit identischen Bezugszeichen versehen und sind teilweise weggelassen, insbesondere wenn bezogen auf eine bestimmte Zeichnung nicht Bezug auf diese Elemente genommen wird. Es versteht sich ferner, dass die gezeigten Ausführungsbeispiele lediglich Möglichkeiten darstellen, die offenbarten erfinderischen Gedanken zu implementieren und keiner Weise einschränkende Wirkung besitzen. Alle Abbildungen umfassen lediglich schematische oder Diagramm-Darstellungen, auch wenn nicht immer explizit darauf hingewiesen wird. Dabei können Details, auf die im Rahmen der Erläuterung nicht eingegangen wird, in einzelnen Darstellungen weggelassen sein.

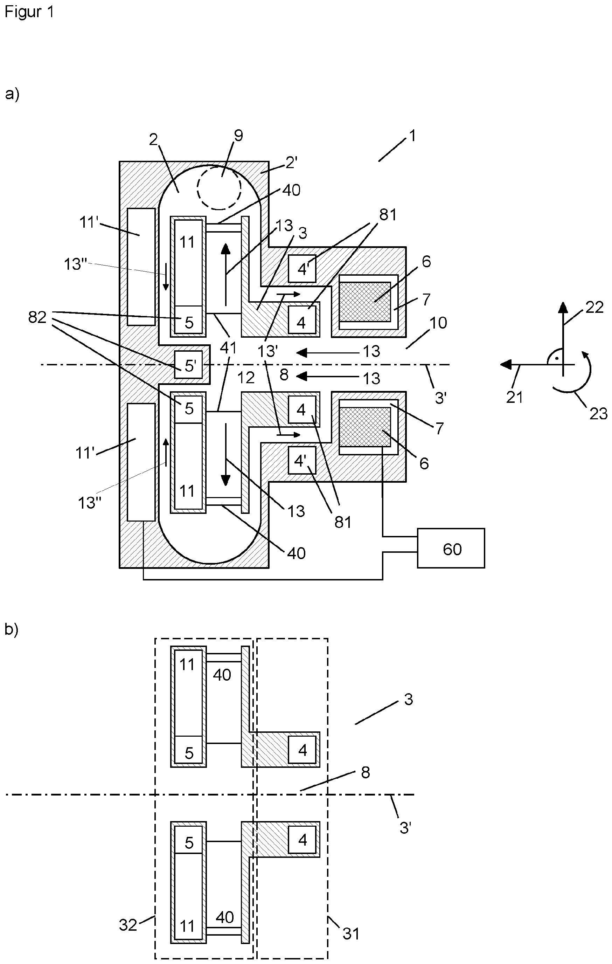

Figur 1a zeigt eine schematische Darstellung einer Pumpe 1, insbesondere einer Pumpe 1 mit einem magnetisch gelagerten Rotor 3 und hierbei bevorzugt einer Blutpumpe 1 mit einem magnetisch gelagerten Rotor 3. Dabei ist die Darstellung in Figur 1a eine Querschnittsansicht. Die Pumpe 1 weist eine Pumpenkammer 2 auf, die von einer Wandung 2' begrenzt wird. Das Material, aus dem die Wandung 2' aufgebaut ist, kann beispielsweise ein Metall sein, zum Beispiel Stahl, insbesondere auch ein Edelstahl, oder auch ein Titan, beispielsweise auch eine Titanlegierung oder auch eine Keramik. In bestimmten Anwendungen kann das Material der Wandung 2' aber beispielsweise auch ein Kunststoff oder zum Beispiel eine Legierung oder eine Kombination aus einigen oder mehreren der vorgenannten Materialien sein. Die Wandung 2' kann beispielsweise mehrstückig hergestellt und beispielsweise während der Herstellung der Pumpe 1 zusammengesetzt werden. Die Pumpenkammer 2 besitzt einen Einlass 10 und einen Auslass 9 und eine Fluidverbindung 12, das heißt, ein Volumen, in dem Fluid strömen kann, zwischen dem Einlass 10 und dem Auslass 9. Der Einlass 10 ist eine Öffnung in der Wandung 2', die einer Zufuhr von Fluid in die Pumpenkammer 2 dient und der Auslass 9 ist eine Öffnung in der Wandung 2', die der Abfuhr von Fluid aus der Pumpenkammer 2 dient. Als Fluid kommen beispielsweise Flüssigkeiten oder Gase in Betracht. Insbesondere kann es sich bei dem Fluid um Blut handeln.

Die Pumpe 1 in Figur 1a weist ferner einen Rotor 3 auf, der ausgebildet ist, sich um eine Rotationsachse 3' zu drehen. Die Rotationsachse 3' kann beispielsweise in einer mittigen Position des Einlasses 10 angeordnet, also am Einlass 10 ausgerichtet sein. Wie vorstehend erläutert wird angenommen, dass der Rotor nur geringe translatorische Bewegungen ausführt, so dass, mit dem Ziel einer einfacheren und verständlicheren Beschreibung, die Rotationsachse bezüglich der Pumpenkammer als feststehend angesehen werden kann.

Die Rotationsachse 3' kann gleichzeitig auch eine Art Symmetrieachse für die Pumpe 1 darstellen, deren in Figur 1a dargestellte Komponenten im Wesentlichen symmetrisch zu dieser angeordnet sind. Die Pumpe 1 und auch der Rotor 3 können eine im Wesentlichen, bezüglich der Rotationsachse 3', radialsymmetrische Gestalt und Anordnung aufweisen. Im Wesentlichen bedeutet, dass lokal auch von der Symmetrie abgewichen werden kann.

Die Rotationsachse 3' definiert eine axiale Richtung 21, die in einem Koordinatensystem als axiale Achse 21 angegeben werden kann. Senkrecht zu der axialen Richtung 21 kann eine radiale Richtung 22 festgelegt und in dem Koordinatensystem als radiale Achse 22 eingetragen werden. Eine Drehrichtung mit einer Drehachse senkrecht zur axialen Achse 21 kann als eine Kipprichtung 23 angeben werden.

Wie in Figur 1a dargestellt, kann ein Fluidfluss 13, also ein Fluss von Fluid, beispielsweise Blut, vom Einlass 10 weg durch die Fluidverbindung 12 zunächst in der axialen Richtung 21 geführt werden. Im Weiteren kann der Fluidfluss 13 zum Auslass 9 beispielsweise in der radialen Richtung 22 geleitet werden. Das Fluid kann die Pumpenkammer 2 schließlich über den Auslass 9 verlassen. Dabei besteht die Möglichkeit, dass der Fluidfluss 13 am Auslass 9 im Wesentlichen keine Komponente, d.h. keine Komponente einer Fließrichtung, in der axialen Richtung 21 aufweist.

Neben dem Fluidfluss 13, der einen Hauptfluss von Fluid durch die Pumpe 1 darstellt, was in Figur 1a durch große Pfeile gekennzeichnet ist, kann ein sekundärer Fluidfluss 13', beispielsweise ein sekundärer Blutfluss 13', existieren, wobei bei dem sekundären Fluidfluss 13' weniger Fluid fließt als bei dem Fluidfluss 13, was durch kleine Pfeile in Figur 1a symbolisiert ist. Der sekundäre Fluidfluss 13' kann beispielsweise zwischen Rotor 3 und Wandung 2', beispielsweise zwischen einem erstem Rotormagneten 4 und der Wandung 2' geführt sein, in diesem Fall zum Beispiel in der axialen Richtung 21 zum Einlass 10 hin.

Darüber hinaus kann ein tertiärer Fluidfluss 13" existieren, wobei bei dem tertiären Fluidfluss 13" weniger Fluid fließt als bei dem Fluidfluss 13, was durch kleine Pfeile in Figur 1a symbolisiert ist. Der tertiäre Fluidfluss 13" kann beispielsweise zwischen Rotor 3 und Wandung 2', beispielsweise zwischen einem Motormagneten 11 und der Wandung 2' geführt sein, in diesem Fall zum Beispiel in der radialen Richtung 22 zur Rotationsachse 3' hin.

Sekundärer Fluidfluss 13' und tertiärer Fluidfluss 13" können jeweils eine andere Fließrichtung aufweisen und können helfen, einen Raum zwischen Rotor 3 und Wandung 2' mit Fluid zu füllen und das Fluid dort in Bewegung zu halten. Dies kann beispielsweise dazu dienen, das Risiko einer Verklumpung des Fluids oder, beispielsweise, im Falle von Blut, das Risiko einer Thrombose zu vermindern.

Ferner zeigt Figur 1a auch einen Impeller 40. Der Impeller 40 kann beispielsweise Flügel und/oder Schaufeln aufweisen, die ausgebildet sind, den Fluidfluss 13, insbesondere bei Drehung des Impellers 40, anzutreiben. Auch der sekundäre Fluidfluss 13' und der tertiäre Fluidfluss 13" können jeweils so angetrieben werden. Der Impeller 40 ist starr mit dem Rotor 3 gekoppelt und dreht sich bei Drehung des Rotors 3 mit dem Rotor 3 mit. Der Impeller 40 hat in Figur 1a eine im Wesentlichen scheibenförmige Gestalt. Das heißt, seine Ausdehnung in axialer Richtung 21 ist kleiner als in der radialen Richtung 22, wobei eine gedachte Einhüllende (nicht dargestellt) des Impellers 40 im Wesentlichen scheibenförmig, also flach ist. Denkbar ist auch eine halbaxiale Impeller-Anordnung, bei der ein axialer Anteil enthalten ist und die Strömungsrichtung nicht sprunghaft von im Wesentlichen axial zu im Wesentlichen nicht axial überführt wird.

Angrenzend an den Impeller 40 ist der Motormagnet 11 angeordnet. Der Motormagnet 11 kann mehrere Motormagneten einschließen. Der Motormagnet 11 kann ein Permanentmagnet sein, beispielsweise ein SeltenErdmagnet, z.B. NdFeB oder SmCo. Darüber hinaus kann eine Magnetisierungsrichtung des Motormagneten 11 in der axialen Richtung 21 verlaufen. Bei mehreren Motormagneten 11 besteht die Möglichkeit, dass benachbarte Motormagneten 11 eine jeweils zueinander verschiedene Magnetisierungsrichtung aufweisen. Andere Magnetisierungsrichtungen, beispielsweise eine tangentiale oder eine radiale Magnetisierungsrichtung, sind ebenfalls denkbar. Ebenfalls ist denkbar, dass die Motormagnete in den Schaufeln des Impellers sitzen.

Dem Motormagnet 11 gegenüberliegend ist der Motorstator 11' dargestellt. Der Motorstator 11' kann eine oder mehrere Motorspulen umfassen (nicht gezeigt). Darüber hinaus ist der Motorstator 11' an der Wandung 2' angeordnet, beispielsweise in die Wandung 2' integriert oder, von der Pumpenkammer 2 ausgehend, hinter der Wandung 2' positioniert. Der Motorstator 11' ist dazu ausgebildet, mit den Motorspulen, die von einem elektrischen Strom durchflossen sein können, ein Magnetfeld zu erzeugen, das beispielsweise eine Komponente in der axialen Richtung 21 aufweisen kann, und das ferner ausgebildet, mit dem Magnetfeld der Motormagneten 11 zusammenzuwirken, um den Impeller 40 rotatorisch anzutreiben. Der Impeller 40 kann sich in einem solchen Fall um die Rotationsachse 3' drehen, beispielsweise zusammen mit dem Rotor 3.

In Figur 1a ist auch der erste Rotormagnet 4 dargestellt. Der erste Rotormagnet 4 kann beispielsweise ein Permanentmagnet sein und er kann ringförmig in einem Abstand um die Rotationsachse 3' umlaufend im oder auch am Rotor 3 angeordnet sein. Die Magnetisierungsrichtung des ersten Rotormagneten 4 kann beispielsweise in der radialen 22 oder in der axialen Richtung 21 bereitgestellt sein. Er kann auch in ein oder mehrere Abschnitte, beispielsweise in der axialen Richtung 21, mit jeweils eigener Magnetisierung unterteilt sein. Die Magnetisierungsrichtung der verschiedenen Abschnitte kann voneinander abweichen, beispielsweise auch entgegengesetzt sein. Dabei sind prinzipiell auch andere Anordnungen denkbar, wie beispielsweise eine Anordnung als ringförmig gestaltetes Halbach-Array, bei dem die Magnetisierungsrichtung einzelner Abschnitte des Halbach-Arrays in Bezug zueinander wechseln kann.

Zum ersten Rotormagnetenen 4 radial beabstandet und diesem gegenüberliegend ist an der Wandung 2' ein erster Kammermagnet 4' angeordnet. Der erste Kammermagnet 4' ist in der Nähe einer Steuerspule 6 angeordnet. Der erste Kammermagnet 4' kann ebenfalls ringförmig in einem Abstand um die Rotationsachse 3' umlaufend angeordnet sein. Die Magnetisierungsrichtung des ersten Kammermagneten 4' kann beispielsweise in der radialen Richtung 22 oder in der axialen Richtung 21 bereitgestellt sein. Er kann auch in ein oder mehrere Abschnitte, beispielsweise in der axialen Richtung 21, mit jeweils eigener Magnetisierung unterteilt sein. Die Magnetisierungsrichtung der verschiedenen Abschnitte kann voneinander abweichen, beispielsweise auch entgegengesetzt sein. Dabei sind prinzipiell auch andere Anordnungen denkbar, wie beispielsweise eine Anordnung als ringförmig gestaltetes Halbach-Array, bei dem die Magnetisierungsrichtung einzelner Abschnitte des Halbach-Arrays in Bezug zueinander wechseln kann. Der Kammermagnet kann auch axial zum Rotormagneten beabstandet sein, wodurch an diesem Lager eine axiale Kraft entsteht. Die Kraft kann z. B. der Anziehungskraft der flussleitenden Elemente 7 um die Steuerspule 6 quasistatisch entgegenwirken oder aber diese Anziehungskraft verstärken.

Die Magnetisierung des ersten Rotormagneten 4 und des ersten Kammermagneten 4' erfolgt in diesem Beispiel so, dass sich beide voneinander abstoßen.

In Figur 1a bilden der erste Rotormagnet 4 und der erste Kammermagnet 4' zusammen ein erstes Magnetlager 81. Das erste Magnetlager 81 ist in dem in Figur 1a gezeigten Fall ein Permanentmagnetlager bzw. ein passives Magnetlager.

In Figur 1a ist ferner ein zweiter Rotormagnet 5 dargestellt. Der zweite Rotormagnet 5 kann beispielsweise ein Permanentmagnet sein und er kann ringförmig in einem Abstand um die Rotationsachse 3' umlaufend an einem Innenumfang 41 des Impellers 40 angeordnet sein. Die Magnetisierungsrichtung des zweiten Rotormagneten 5 kann beispielsweise in der radialen Richtung 22 oder in der axialen Richtung 21 bereitgestellt sein. Er kann auch in ein oder mehrere Abschnitte, beispielsweise in der axialen Richtung 21, mit jeweils eigener Magnetisierung unterteilt sein. Die Magnetisierungsrichtung der verschiedenen Abschnitte kann voneinander abweichen, beispielsweise auch entgegengesetzt sein. Dabei sind prinzipiell auch andere Anordnungen denkbar, wie beispielsweise eine Anordnung als ringförmig gestaltetes Halbach-Array, bei dem die Magnetisierungsrichtung einzelner Abschnitte des Halbach-Arrays in Bezug zueinander wechseln kann.

Dem zweiten Rotormagneten 5 gegenüberliegend an der Wandung 2' ist ein zweiter Kammermagnet 5' angeordnet. Der zweite Kammermagnet 5' kann beispielsweise ebenfalls ringförmig oder, beispielsweise, auch zylindrisch sein. Es ist möglich, den zweiten Kammermagneten 5' so anzuordnen, dass die Rotationsachse 3' durch den zweiten Kammermagneten 5' hindurch verläuft. Dabei kann die Rotationsachse 3' an einer Zylinderachse des zweiten Kammermagneten 5' ausgerichtet sein, insbesondere wenn der zweite Kammermagnet 5' eine zylindrische oder eine ringförmige Gestalt, beispielsweise in Form eines Hohlzylinders, hat. Die Magnetisierungsrichtung des zweiten Kammermagneten 5' kann beispielsweise in der radialen Richtung 22 oder der axialen Richtung 21 bereitgestellt sein. Er kann auch in ein oder mehrere Abschnitte, beispielsweise in der axialen Richtung 21, mit jeweils eigener Magnetisierung unterteilt sein. Die Magnetisierungsrichtung der verschiedenen Abschnitte kann voneinander abweichen, beispielsweise auch entgegengesetzt sein. Dabei sind prinzipiell auch andere Anordnungen denkbar, wie beispielsweise eine Anordnung als ringförmig gestaltetes Halbach-Array, bei dem die Magnetisierungsrichtung einzelner Abschnitte des Halbach-Arrays in Bezug zueinander wechseln kann.

Die Magnetisierung des zweiten Rotormagneten 5 und des zweiten Kammermagneten 5' erfolgt in diesem Beispiel so, dass sich beide voneinander abstoßen.

Erster Rotormagnet 4, zweiter Rotormagnet 5, erster Kammermagnet 4' und zweiter Kammermagnet 5' können beispielsweise mit Neodymmagneten realisiert werden.

Der zweite Rotormagnet 5 und der zweite Kammermagnet 5' bilden in diesem Beispiel zusammen ein zweites Magnetlager 82. Das zweite Magnetlager ist in dem in Figur 1a gezeigten Fall ein Permanentmagnetlager bzw. ein passives Magnetlager.

Figur 1a zeigt auch die Steuerspule 6. Die Steuerspule 6 ist in der gezeigten Darstellung vollständig zwischen, in der axialen Richtung 21 betrachtet, dem Einlass 10 und dem Rotor 3 angeordnet. Eine Windung einer Wicklung der Steuerspule 6 ist, im Abstand von der Rotationsachse 3', also um die axiale Richtung 21 herum, um die Fluidverbindung 12 und damit um die Pumpenkammer 2 herumgeführt. Ein mit der Steuerspule 6 erzeugbares Magnetfeld kann eine Komponente in der axialen Richtung 21 aufweisen. Das Magnetfeld der Steuerspule 6 ist auch dazu ausgebildet mit einem Magnetfeld des ersten Rotormagneten 4 zusammenzuwirken und eine Axialkraft auf den Rotor in der axialen Richtung 21 auszuüben. Das Magnetfeld der Steuerspule 6 kann mit einem Steuerstrom erzeugt werden. Die Axialkraft auf den Rotor 3 kann, beispielsweise mit dem Steuerstrom, steuerbar sein.

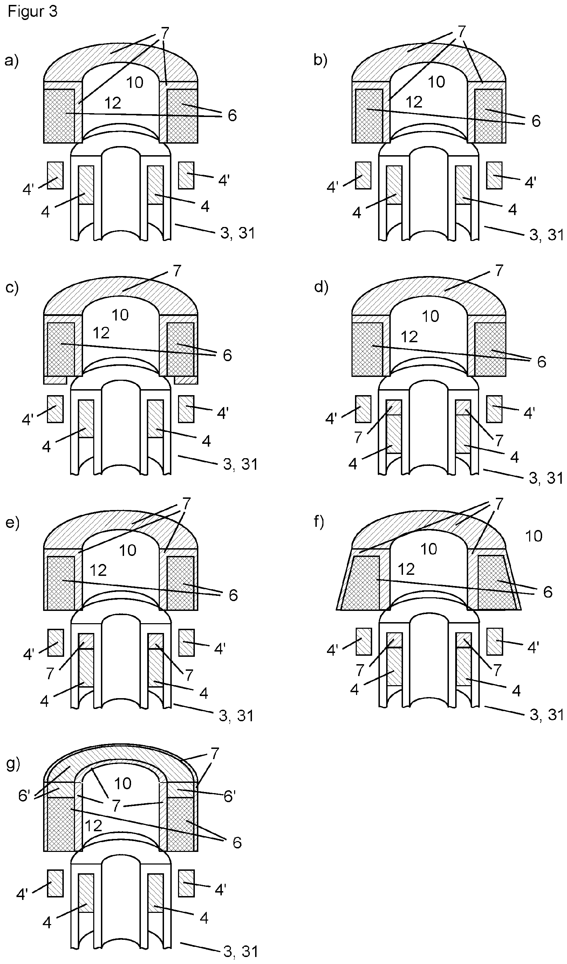

Benachbart zur Steuerspule 6 ist in Figur 1a auch ein magnetisches Material 7 gezeigt. Das magnetische Material 7 dient der Führung des von der Steuerspule erzeugten Magnetfeldes und führt einen damit verbundenen magnetischen Fluss, so dass eine höhere Axialkraft als ohne das magnetische Material 7 erzeugt werden kann. Auf weitere Details der Anordnung des magnetischen Materials 7 wird im Rahmen der Diskussion zu Figur 3 tiefer eingegangen. Das magnetische Material 7 kann in die Wandung 2' integriert sein und/oder einen Teil der Wandung 2' bilden und/oder auch an der Wandung 2' angeordnet sein.

Zusätzlich zeigt Figur 1a auch eine Steuereinheit 60. Die Steuereinheit 60 kann einen Steuerstrom für die Steuerspule 6 bereitstellen. Mit dem Steuerstrom kann beispielsweise eine Stärke des Magnetfeldes der Steuerspule 6 gesteuert werden. Damit kann schließlich die Axialkraft auf den Rotor 3 gesteuert werden, die im Zusammenwirken des von der Steuerspule 6 erzeugten Magnetfeldes mit dem Magnetfeld des ersten Rotormagneten 4 erzeugt werden kann. Der Steuerstrom kann so bereitgestellt werden, dass der Rotor 3 in einer von der Wandung 2' der Pumpenkammer 2 axial beabstandeten Position gehalten wird, beispielsweise indem der Steuerstrom jeweils so groß ist, dass damit jeweils eine Axialkraft erzeugt wird, die für das Halten in der axial beabstandeten Position notwendig ist. Als Kräfte auf den Rotor können beispielsweise Kräfte sein, die auf ein Wirken der Magnetfelder des ersten Rotormagneten 4, des zweiten Rotormagneten 5, des ersten Kammermagneten 4' und des zweiten Kammermagneten 5' zurückzuführen sind und/oder auch externe Kräfte auf den Rotor 3, beispielsweise ausgehend vom von dem Fluidfluss 13, einer Gravitation und/oder einer beschleunigten Bewegung der Pumpe 1.

Die Steuereinheit 60 kann beispielsweise als Mikrocontroller, als Digitaler Signalprozessor, als FPGA, oder als ASIC oder auch als eine Kombination davon ausgebildet sein und kann auch weitere elektronische Komponenten enthalten. Dazu können beispielsweise auch Sensoren zählen, wie beispielsweise ein Abstands- oder auch Positionssensor, beispielsweise zur Bestimmung einer Position des Rotors 3, beispielsweise in der axialen Richtung 21. Weitere mögliche Sensoren sind beispielsweise ein Magnetfeldsensor, ein Drehzahlsensor, ein Drucksensor, ein Fluidflusssensor und dergleichen.

Die Steuereinheit 60 kann auch dazu dienen, den Motorstator 11' zu steuern, beispielsweise, in dem die Motorspule des Motorstators 11' durch die Steuereinheit 60 mit einem elektrischen Strom, beispielsweise einem zeitlich veränderlichen elektrischen Strom, versorgt wird. Dieser elektrische Strom kann beispielsweise derart sein, dass damit ein für einen rotatorischen Antrieb der Pumpe 1 geeignetes Magnetfeld erzeugt wird, wobei das Magnetfeld der Motorspule dafür mit dem Magnetfeld des Motormagneten zusammenwirken kann. Der von der Steuereinheit 60 für den Motorstator 11' bereitgestellte Strom kann aber auch noch dafür ausgelegt sein, eine Axialkraft in der axialen Richtung 21 auf den Rotor 3, auch in Zusammenwirken mit dem Motormagneten 11, zu erzeugen. Die Steuereinheit 60 kann zu diesem Zweck einen Regler aufweisen, der beispielsweise nach dem Prinzip einer feldorientierten Regelung arbeitet. Die vom Motorstator 11' mit dem Motormagneten 11 erzeugte Axialkraft kann dabei mit der Erzeugung der Axialkraft durch die Steuerspule 6 mit dem ersten Rotormagneten 4 koordiniert sein. Es ist beispielsweise möglich, die mit dem Motor, umfassend den Motorstator 11' und Motormagneten 11, erzeugte Axialkraft als Vorspannkraft für die von der Steuerspule 6 erzeugte Axialkraft zu gestalten. Beispielsweise kann die Vorspannkraft drehzahlabhängig sein.

Figur 1b zeigt in einer Querschnittsdarstellung den Rotor 3 aus Figur 1a. Darin weist der Rotor 3 einen ersten Abschnitt 31 und einen zweiten Abschnitt 32 auf. Im ersten Abschnitt 31 ist beispielsweise der erste Rotormagnet 4 angeordnet. Im zweiten Abschnitt 32 ist der Impeller 40 gezeigt sowie der Motormagnet 11 und der zweite Rotormagnet 5. Insofern weist der erste Abschnitt 31 das erste Magnetlager 81 auf und der zweite Abschnitt weist das zweite Magnetlager 82 und den Impeller 40 auf. Mit dem ersten und dem zweiten Magnetlager 81, 82 kann der Rotor in der radialen Richtung 22 vollständig gestützt werden. Erstes und zweites Magnetlager 81, 82 sind in axialer Richtung voneinander beabstandet. Damit ist beispielsweise auch eine Bewegung des Rotors 3 in der Kipprichtung 23 zumindest teilweise unterdrückbar.

Der Rotor 3 weist darüber hinaus eine um die Rotationsachse 3' herum angeordnete Ausnehmung 8 (in Figur 1a aus Übersichtlichkeitsgründen nicht gezeigt) auf. Die Ausnehmung 8 kann beispielsweise radialsymmetrisch um die Rotationsachse 3' ausgebildet sein, beispielsweise als Bohrung. Die Ausnehmung 8 bietet den Vorteil, dass das Fluid dort hindurchgeführt werden kann. Somit ist es möglich, dass beispielsweise der Abstand zwischen erstem Rotormagneten 4 und erstem Kammermagneten 4' möglichst klein gehalten werden kann, da das Fluid nicht zwischen erstem Rotormagneten 4 und erstem Kammermagneten 4' geführt werden muss, sondern durch die Ausnehmung 8 strömen kann. Damit können die abstandsabhängigen Magnetkräfte zwischen erstem Rotormagneten 4 und erstem Kammermagneten 4' möglichst groß gestaltet werden. Außerdem können so aber die Magneten selbst klein und somit der Bauraum der Pumpe 1 klein gehalten werden. In der radialen Richtung betrachtet kann ein Querschnitt der Ausnehmung 8 beispielsweise einem Querschnitt des Einlasses 10 bezüglich Form und Fläche entsprechen oder auch im Wesentlichen entsprechen, mit dem Ziel, einen Strömungswiderstand für das Fluid durch die Pumpe 1 gering zu halten. Dabei ist es auch möglich, dass die Querschnitte der Ausnehmung 8 und des Einlasses 10 zueinander ausgerichtet sind, wie dies in Abbildung 1a angedeutet ist.

Sowohl erster Abschnitt 31 als auch zweiter Abschnitt 32 können die Ausnehmung 8 aufweisen und auf diese Weise das Fluid zum Impeller 40 führen, so dass es von dort zum Auslass 9 strömen bzw. vom Impeller 40 dorthin getrieben werden kann.

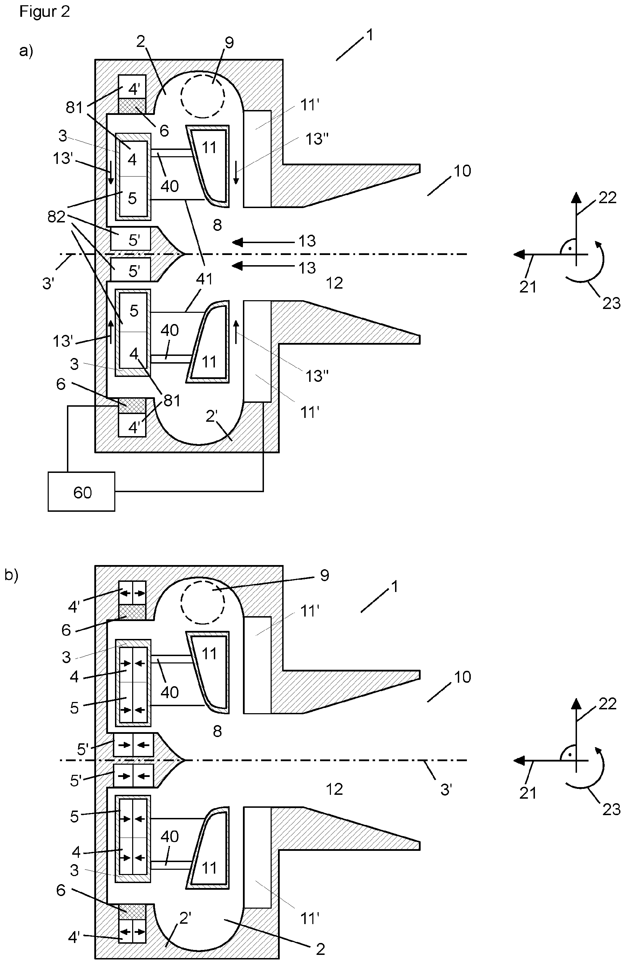

Figur 2a zeigt eine schematische Darstellung einer Pumpe 1 gemäß der vorliegenden Offenbarung, die eine Reihe von Gemeinsamkeiten mit der in Figur 1a dargestellten Pumpe 1 aufweist, auf die hier nicht erneut eingegangen werden soll. Vielmehr werden im Folgenden die Unterschiede zur in Figur 1a dargestellten Pumpe 1 diskutiert. So ist in der in Figur 2a, ebenfalls in einer Querschnittsdarstellung, gezeigten Pumpe 1 der Motorstator 11' an der Pumpenkammer 2 an einer auf einer dem Einlass 10 zugewandten Seite des Impellers 40 angeordnet. In axialer Richtung gegenüberliegend, am Impeller 40, sind die Motormagneten 11 angebracht. Motormagneten 11 und Motorstator 11' unterscheiden sich hier vom in Figur 1a gezeigten Beispiel lediglich bezüglich ihrer Anordnung in der Pumpe 1, nicht jedoch bezüglich ihrer Funktion und den oben beschriebenen weiteren Details.

Darüber hinaus ist, in Figur 2a, der erste Rotormagnet 4 in der Nähe eines Außenumfangs des Impellers 40 angeordnet, in diesem Fall auf einer dem Einlass 10 abgewandten Seite des Impellers 40. Der erste Kammermagnet 4' ist radial beabstandet zum ersten Rotormagneten 4 an der Wandung 2' der Pumpenkammer 2 angeordnet. Dabei grenzt der erste Kammermagnet 4' an die Wicklung der Steuerspule 6 an. Die Wicklung der Steuerspule 6 ist zwischen dem ersten Kammermagneten 4' und der Pumpenkammer 2 angeordnet. Eine Windung der Wicklung ist um die axiale Richtung 21 herum um die Pumpenkammer 2 geführt. Dies ermöglicht eine hohe Effizienz der Steuersteuerspule 6, wobei hiermit eine hohe erzeugbare Kraftwirkung (gemeinsam mit dem ersten Rotormagneten 4) pro Einheit des elektrischen Steuerspulenstroms gemeint ist. Wie gezeigt, ist somit die Steuerspule 6, im Gegensatz zu Figur 1a, nicht in der Nähe des Einlasses 10 angeordnet, sondern in der Nähe des Auslasses bzw. in der Nähe des Impellers. Die Steuerspule 6 kann aber auch hier, zusammen mit dem ersten Rotormagneten 4, eine Kraft in der axialen Richtung 21 auf den Rotor 3 ausüben. Darüber hinaus, aufgrund der Beabstandung des ersten Rotormagneten 4 und der Steuerspule 6 von der Rotationsachse 3', kann die von erstem Rotormagneten 4 und Steuerspule 6 erzeugte Kraft eine Bewegung des Rotors 3 in der Kipprichtung 23 vermindern.

Dies ist zusätzlich auch Aufgabe des ersten Rotormagneten 4 gemeinsam mit dem ersten Kammermagneten 4', deren Magnetfelder wechselwirken und eine Kraft auf den Rotor erzeugen. In diesem Beispiel kann diese Kraft eine Anziehungskraft zwischen erstem Rotormagneten 4 und erstem Kammermagneten 4' sein. Grundsätzlich kann die so erzeugte Kraft auf den Rotor 3 auch in der axialen Richtung 21 und in der Kipprichtung 23 wirken.

Der zweite Rotormagnet 5 und der zweite Kammermagnet 5' ähneln im Wesentlichen den entsprechenden Elementen von Figur 1a. In den Figuren 2a und 2b sind der erste Rotormagnet 4 und der zweite Rotormagnet 5 als getrennte Magneten ausgeführt. Dabei besteht der Vorteil beispielsweise darin, dass die Magnetisierung beider Rotormagneten unterschiedlich nach Polarisierung und/oder Stärke bereitgestellt werden kann und somit die jeweils wirkenden Kräfte im ersten Magnetlager 81 bzw. im zweiten Magnetlager 82 unabhängig voneinander konfiguriert werden können.

Zusätzlich besteht jedoch auch die Möglichkeit, dass der zweite Rotormagnet 5 und der erste Rotormagnet 4 zu einem einzigen Rotormagneten zusammengefasst werden. Dies ist beispielsweise möglich, wenn beide eine entsprechend aufeinander abgestimmt Magnetisierung aufweisen. Dies ist beispielsweise in Figur 2b gezeigt.

In Figur 2b, die auf Figur 2a basiert, weisen die gezeigten Rotor- bzw. Kammermagneten 4, 4', 5, 5' jeweils mehrere Abschnitte in der axialen Richtung 21 auf. Jeder der gezeigten Abschnitte besitzt eine Magnetisierung, dargestellt durch Pfeile in den Darstellungen der jeweiligen Magneten 4, 4', 5, 5', in der axialen Richtung 21, wobei prinzipiell auch jeweils eine Magnetisierung in der radialen Richtung 22 denkbar wäre. Die Magnetisierung in der axialen Richtung 21 erleichtert jedoch Herstellung der Magneten und den Zusammenbau der Pumpe 1. Dabei wird im dargestellten Beispiel aufgrund der jeweiligen Pfeilrichtung deutlich, dass der erste Rotormagnet 4 und der zweite Rotormagnet 5 jeweils gleich magnetisierte Abschnitte in der axialen Richtung 21 aufweisen, womit der erste und der zweite Rotormagnet 4, 5 durch einen einzigen, beispielsweise in die gezeigten axialen Abschnitte unterteilten Magneten ersetzt werden können. Eine alternierende Magnetisierung von ersten Rotormagnet 4 und zweiten Rotormagnet 5 ist ebenfalls denkbar.

Die Figuren 3a bis 3g zeigen schematische Darstellungen des ersten Abschnitts 31 des Rotors 3 und der Steuerspule 6 in einer Querschnittsansicht und zum Teil perspektivischen Ansicht und setzen damit das Beispiel aus den Figuren 1a und 1b fort. Relative Richtungen, wie sie in den Figuren 1a und 1b definiert sind, werden übernommen. Nicht gezeigte Details entsprechen im Wesentlichen den Darstellungen der Figuren 1a und 1b. Insbesondere werden den Figuren 3a bis 3g jeweils der Rotor 3, der erste Rotormagnet 4 sowie der erste Kammermagnet 4', die Steuerspule 6 und das magnetische Material 7 gezeigt.

Figur 3a zeigt eine mögliche Anordnung des magnetisch leitfähigen Materials 7, und zwar jeweils an der Steuerspule 6, wobei das magnetisch leitfähige Material 7 auf der dem Einlass 10 zugewandten Seite des Steuerspule 6 sowie auf der der Fluidverbindung 12 zugewandten Seite der Steuerspule 6, also zwischen Fluidverbindung 12 und Steuerspule 6 platziert, und hier auch an die Wicklung der Steuerspule 6 angrenzend platziert ist. Windungen einer Wicklung der Steuerspule sind um die Fluidverbindung 12 herumgeführt. Eine Windung der Wicklung ist damit auch um die axiale Richtung 21 herum geführt. Die der Fluidverbindung 12 abgewandte Seite der Steuerspule 6 sowie die dem Rotor 3 zugewandte Seite der Steuerspule 6 sind nicht mit dem magnetisch leitfähigen Material 7 versehen. Das von der Steuerspule 6 erzeugte Magnetfeld kann bevorzugt im magnetisch leitfähigen Material 7 geleitet werden, womit die magnetische Flussdichte im Spalt zum Rotormagneten 4 verstärkt wird. Das heißt, bei gleichem Steuerstrom kann eine größere magnetische lokale Flussdichte und damit auch eine größere Axialkraft zwischen Steuerspule 6 und Rotor 3 erzeugt werden. Dies ist beispielsweise in Figur 3a an der Stelle der Fall, wo das magnetisch leitfähige Material 7 dem Rotor 3 bzw. hier auch dem ersten Rotormagneten 4 am nächsten kommt. In diesem Bereich ist auch die axiale Kraftwirkung am größten.

Die Vorteile dieser Anordnung des magnetisch leitfähigen Materials 7 bestehen darin, bei gegebenem Steuerstrom im Spalt zum Rotor 3 eine größere magnetische Flussdichte zu erhalten als ohne magnetisch leitfähiges Material 7, so dass die gewünschte axiale Kraftwirkung zwischen Steuerspule 6 und Rotor 3 an der gewünschten Stelle erhöht werden kann. Ein magnetisch leitfähiges Material 7 kann beispielsweise, wie vorstehend erläutert, eine hohe relative magnetische Permeabilität aufweisen wie zum Beispiel ein ferromagnetischer Stoff, beispielsweise Eisen. Typische Werte für die relative magnetische Permeabilität ferromagnetischer Stoffe können beispielsweise im Bereich zwischen 300 und 10000 liegen, wobei für bestimmte Stoffe, wie beispielsweise Mumetalle, amorphe Metalle oder auch nanokristalline Metalle auch Werte deutlich darüber bekannt sind, bis beispielsweise bis 150000 oder sogar bis 500000. Bei bestimmten Stoffen sind auch geringere relative magnetische Permeabilitäten bekannt, die beispielsweise bei Ferriten bis etwa auf 4 absinken können.

Figur 3a zeigt auch einen Versatz zwischen dem ersten Rotormagneten 4 und dem ersten Kammermagneten 4' in der axialen Richtung 21. Mit einem solchen Versatz ist es beispielsweise möglich, eine axiale Vorspannkraft, die beispielsweise vom Einlass 10 weggerichtet ist, zu erzeugen. Dies kann vorteilhaft dazu genutzt werden, den Betrag der von der Steuerspule 6 und dem ersten Rotormagneten 4 bereitgestellten stationären Kraft in der axialen Richtung zu reduzieren und beispielsweise so die Effizienz der Pumpe 1 zu erhöhen.

Figur 3b zeigt eine Variante von Figur 3a, wobei hier zusätzlich zu Figur 3a das magnetisch leitfähige Material 7 auch an der der Fluidverbindung 12 abgewandten Seite der Steuerspule 6 platziert ist. Das magnetisch leitfähige Material 7 kann dabei auf der der Fluidverbindung 12 zugewandten Seite der Steuerspule 6 beispielsweise eine andere Materialdicke in der radialen Richtung 22 aufweisen als an der der Fluidverbindung 12 abgewandten Seite der Steuerspule 6. Beispielsweise, wie hier gezeigt, kann (in der radialen Richtung 22 betrachtet) die Materialdicke des magnetisch leitfähigen Materials 7 an der der Fluidverbindung 12 zugewandten Seite der Steuerspule 6 größer sein als auf der der Fluidverbindung 12 abgewandten Seite der Steuerspule 6. Das Verhältnis der Materialdicken kann beispielsweise so sein, das eine Querschnittsfläche des magnetisch leitfähigen Materials 7 an der der Fluidverbindung zugewandten Seite der Steuerspule 6 in etwa einer Querschnittsfläche des magnetisch leitfähigen Materials 7 an der der Fluidverbindung abgewandten Seite der Steuerspule gleicht, wobei die Querschnittsfläche senkrecht zu der axialen Richtung betrachtet wird. Der Vorteil besteht dabei in einer möglichst einheitlichen magnetischen Materialausnutzung bezüglich einer materialspezifischen zu berücksichtigenden maximalen magnetischen Flussdichte.

Aufbauend auf Figur 3b zeigt Figur 3c eine weitere Variante für die Anordnung des magnetisch leitfähigen Materials 7. In Figur 3c ist zusätzlich zur Anordnung in Figur 3b auch magnetisch leitfähiges Material 7 auf der dem Rotor 3 zugewandten Seite der Steuerspule 6 angeordnet. Dabei kann das magnetisch leitfähige Material 7 so angeordnet sein, dass sich der Spalt zwischen Rotor 3 und Steuerspule 6 im Vergleich zum Beispiel in Figur 3b nicht vergrößert, beispielsweise in dem nur ein Teil der dem Rotor 3 zugewandten Seite der Steuerspule 6 mit magnetisch leitfähigem Material 7 versehen ist. Beispielsweise ist ein Abschnitt der dem Rotor 3 zugewandten Seite der Steuerspule 6, der dem Rotor 3 direkt gegenübersteht, frei von magnetisch leitfähigem Material 7. Mit dieser Anordnung kann die Kraftwirkung weiter fokussiert werden und es besteht auch die Möglichkeit, den magnetischen Fluss, insbesondere im Spalt zwischen Rotor 3 und Steuerspule 6, im Vergleich zur Anordnung in Figur 3c weiter zu erhöhen.

Figur 3d zeigt eine weitere Anordnung des magnetisch leitfähigen Materials 7 analog zu Figur 3a. Zusätzlich zur Anordnung in Figur 3a gibt es hier jedoch einen Abschnitt des magnetisch leitfähigen Materials 7, der im Rotor 3 an einer dem Einlass 10 zugewandten Seite und angrenzend an den ersten Rotormagneten 4 positioniert ist. In diesem Falle kann das magnetisch leitfähige Material 7 beispielsweise als Eisenjoch ausgebildet sein und beispielsweise den magnetischen Fluss weiter konzentrieren.

Figur 3e zeigt beispielhaft eine Kombination der Beispiele aus den Figuren 3b und 3d. Dabei wird deutlich, dass die vorstehenden Beispiele zur Anordnung des magnetisch leitfähigen Materials 7 beliebig kombinierbar sind. Dies trifft in gleicher Weise auch Figur 3f, die auf Figur 3e basiert, zu, wo die Steuerspule 6 im Vergleich zu den vorangegangenen Beispielen die äußere Gestalt eines Kegelstumpfes aufweist. Dies zeigt unter anderem, dass die Steuerspule 6 mit verschiedenen Geometrien verwirklicht werden kann, die auch nicht gezeigte Geometrien einschließt wie beispielsweise eine quaderförmige Geometrie oder auch allgemein eine zylindrische Geometrie, die auch eine ringförmige Geometrie einschließt, mit einer Polygon-Grundfläche.

Ein weiteres Beispiel, wieder mit einer im Wesentlichen zylindrischen Geometrie, ist in Figur 3g gezeigt. Das in Figur 3g gezeigte Beispiel basiert auf dem Beispiel in Figur 3b, wobei an der dem Einlass 10 zugewandten Seite der Steuerspule 6 magnetisch leitfähiges Material 7 angeordnet ist. Stattdessen ist dort jedoch, angrenzend an die Steuerspule 6, ein Vorspannmagnet 6' angeordnet, beispielsweise ein Permanentmagnet. Im Beispiel von Figur 3g grenzt das magnetisch leitfähige Material 7 an den Vorspannmagneten 6' in der radialen Richtung 22 an einem Innen- und einem Außenumfang des in diesem Beispiel ringförmigen Vorspannmagneten 6' an. Die Magnetisierung des Vorspannmagneten 6' kann hier, beispielsweise, in der radialen Richtung 22 erfolgen. Ausgeführt als Permanentmagnet kann der Vorspannmagnet 6' beispielsweise für einen magnetischen Fluss sorgen, ohne dass ein elektrischer Steuerstrom durch die Wicklung der Steuerspule 6 benötigt wird. Damit kann beispielsweise ein Arbeitspunkt von Steuerspule 6 zusammen mit dem ersten Rotormagneten 4 bezüglich des magnetischen Flusses und/oder der axialen Kraft eingestellt werden.

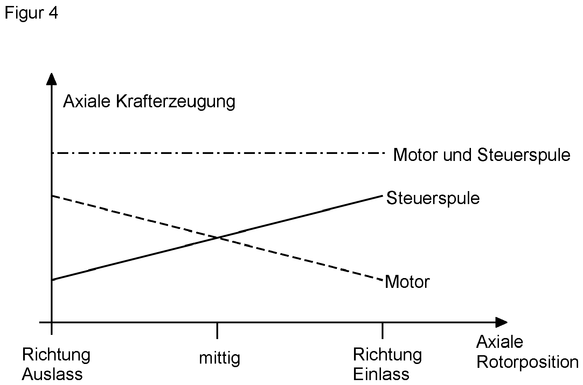

Figur 4 zeigt ein Diagramm für eine Möglichkeit zur axialen Krafterzeugung in Abhängigkeit von einer axialen Position des Rotors 3 und bezieht sich vorwiegend auf das Beispiel in den Figuren 1a und 1b, kann aber in analoger Weise auf das Beispiel in den Figuren 2a und 2b angepasst werden. Dabei wird die Möglichkeit betrachtet, dass sowohl mit dem Motor, der den Motorstator 11' mit der Motorspule und den Motormagneten 11 umfasst, als auch mit der Steuerspule 6, wenn sie mit dem erstem Rotormagneten 4 zusammenwirkt, eine axiale Kraft, die in der axialen Richtung 21 wirkt, auf den Rotor 3 erzeugt werden kann. Motor 11, 11' und Steuerspule 6 mit erstem Rotormagneten 4 können in ihrer Funktion so aufeinander abgestimmt werden, dass die gesamt erzeugte axiale Kraft im Wesentlichen konstant ist. Dies ist mit der konstanten horizontalen Strich-Punkt-Linie in Figur 4 symbolisiert. Dies ist insofern vorteilhaft, da auf diese Weise die axiale Position des Rotors 3 einfacher geregelt werden kann.

Die durchgezogene, ansteigende Linie im Diagramm von Figur 4 betrifft die von der Steuerspule 6 zusammen mit dem ersten Rotormagneten 4 erzeugte Kraft in der axialen Richtung. Sie nimmt zu (bei gleichem Steuerstrom) je weiter sich eine axiale Position des Rotors 3 dem Einlass 10 nähert.

Die gestrichelte, abfallende Linie im Diagramm von Figur 4 betrifft die vom Motor erzeugte Kraft in der axialen Richtung. Sie nimmt ab (bei sonst gleicher Motoransteuerung) je weiter sich eine axiale Position des Rotors 3 dem Einlass 10 nähert.

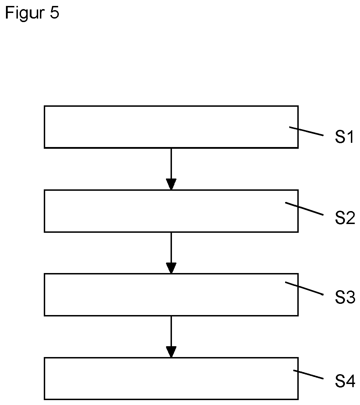

Figur 5 zeigt ein Ablaufdiagramm für ein Verfahren zum Betreiben einer Pumpe 1. Das Verfahren umfasst die Schritte:
- S1: Bereitstellen eines Motormagnetfeldes zum Antreiben eines mit einem Rotor 3 verbundenen Impellers 40 zum Treiben eines Fluidflusses 13 von einem Einlass 10 zu einem Auslass 9 einer Pumpenkammer 2;
- S2: Bereitstellen, mit einer Steuerspule 6 und einem am Rotor 3 angeordneten ersten Rotormagneten 4, einer steuerbaren Axialkraft auf den Rotor 3 in Richtung einer Rotationsachse 3' des Rotors 3, die eine axiale Richtung 21 definiert;
- S3: Bereitstellen eines Steuerstroms für die Steuerspule 6, um den Rotor 3 in einer von einer Wandung 2' der Pumpenkammer 2 axial beabstandeten Position zu halten, vorzugsweise in einer zeitlich veränderlichen Position, bei der sich die Summe aller Kräfte auf den Rotor 3 außer der steuerbaren Axialkraft in der axialen Richtung 21 zu null addieren;
- S4: Bereitstellen einer Kraft zwischen dem ersten Rotormagneten 4 und einem an der Pumpenkammer 2 nahe einer Wicklung der Steuerspule 6 angeordneten ersten Kammermagneten 4'.

Die genannten Schritte können in dieser Reihenfolge, können aber auch in einer anderen Reihenfolge durchgeführt werden. Einzelne oder auch mehrere der Schritte können auch mehrfach ausgeführt werden.

Im Rahmen des Verfahrens kann eine Nullkraftregelung zum Einsatz kommen, bei der ein dem Betrage nach besonders kleiner Steuerstrom benötigt wird, da der Rotor 3 in der gegebenenfalls zeitlich veränderlichen Position gehalten wird, bei der sich die Summe aller Kräfte auf den Rotor 3 außer der steuerbaren Axialkraft in der axialen Richtung 21 zu null addieren. Diese ermöglicht einen effizienten Betrieb der Pumpe 1 mit minimierter elektrischer Steuerleistung.

### Liste der Bezugszeichen

- 1: Pumpe
- 2: Pumpenkammer
- 2': Wandung
- 3: Rotor
- 3': Rotationsachse
- 4: erster Rotormagnet
- 4': erster Kammermagnet
- 5: zweiter Rotormagnet
- 5: zweiter Kammermagnet
- 6: Steuerspule
- 6': Vorspannmagnet
- 7: magnetisch leitfähiges Material
- 8: Ausnehmung
- 9: Auslass
- 10: Einlass
- 11': Motorstator
- 11: Motormagnet
- 12: Fluidverbindung
- 13: Fluidfluss
- 13': sekundärer Fluidfluss
- 13": tertiärer Fluidfluss
- 21: axiale Richtung
- 22: radiale Richtung
- 23: Kipprichtung
- 31: erster Abschnitt (des Rotors)
- 32: zweiter Abschnitt (des Rotors)
- 40: Impeller
- 41: Innenumfang des Impellers
- 60: Steuereinheit
- 81: erstes Magnetlager
- 82: zweites Magnetlager

## Patentansprüche

1. Pumpe (1), insbesondere eine Blutpumpe, umfassend
- eine Pumpenkammer (2) mit einer Wandung (2') und einer Fluidverbindung (12) für einen Fluidfluss (13), insbesondere einen Blutfluss, zwischen einem Einlass (10) und einem Auslass (9) der Pumpenkammer (2);
- einen in der Pumpenkammer (2) angeordneten, mit einem Rotor (3) verbundenen Impeller (40), ausgebildet zum Drehen um eine Rotationsachse (3'), die am Einlass (10) ausgerichtet ist und eine axiale Richtung (21) definiert, und ausgebildet, den Fluidfluss (13) vom Einlass (10) zum Auslass (9) zu treiben;
- einen an der Pumpenkammer (2) angeordneten Motorstator (11'), wobei der Motorstator (11') mindestens eine Motorspule umfasst, die ausgebildet ist, ein Motormagnetfeld bereitzustellen, das ausgebildet ist, mit einem Magnetfeld eines am Impeller (40) angeordneten Motormagneten (11) zusammenzuwirken, um den Impeller (40) rotatorisch anzutreiben;
- eine an der Pumpenkammer (2) angeordnete Steuerspule (6), wobei eine oder mehrere Windungen einer Wicklung der Steuerspule (6) an der Wandung (2') um die Pumpenkammer (2) geführt sind und die Steuerspule (6) ausgebildet ist, mit einem Steuerstrom ein Magnetfeld zu erzeugen, das ausgebildet ist, mit einem am Rotor (3) angeordneten ersten Rotormagneten (4) eine steuerbare Axialkraft auf den Rotor (3) in der axialen Richtung (21) auszuüben;
- eine Steuereinheit (60), dazu ausgebildet, den Steuerstrom für die Steuerspule (6) bereitzustellen, um den Rotor (3) in einer von der Wandung (2') der Pumpenkammer (2) axial beabstandeten Position zu halten;
- einen ersten Kammermagneten (4'), der an der Pumpenkammer (2), nahe der Wicklung der Steuerspule (6) und dem ersten Rotormagneten (4) gegenüberliegend angeordnet und ausgebildet ist, zusammen mit dem ersten Rotormagneten (4) eine Kraft zwischen dem ersten Rotormagneten (4) und dem ersten Kammermagneten (4') bereitzustellen, um den Rotor (3) relativ zur Wandung (2') der Pumpenkammer (2) auszurichten.

2. Pumpe (1) nach Anspruch 1, mit einem zweiten Magnetlager (82), umfassend einen zweiten Kammermagneten (5'), der an der Pumpenkammer (2) dort angeordnet ist, wo die Rotationsachse (3') auf die Wandung (2') trifft, und einen dem zweiten Kammermagneten (5') gegenüberliegend am Rotor (3) angeordneten zweiten Rotormagneten (5), wobei das zweite Magnetlager (82) ausgebildet ist, eine Abstoßungskraft zwischen dem zweiten Rotormagneten (5) und dem zweiten Kammermagneten (5') bereitzustellen, um den Rotor (3) oder einen Abschnitt des Rotors (3) in einer von der Wandung (2') der Pumpenkammer (2) in einer radialen Richtung (22), die senkrecht zu der axialen Richtung (21) definiert ist, beabstandeten Position zu halten.

3. Pumpe (1) nach einem der Ansprüche 1 bis 2, wobei der Rotor (3) um die Rotationsachse (3') eine Ausnehmung (8) aufweist und dazu ausgebildet ist, den Fluidfluss (13) durch die Ausnehmung (8) zu führen.

4. Pumpe (1) nach einem der Ansprüche 1 bis 3, wobei die Kraft zwischen dem ersten Rotormagneten (4) und dem ersten Kammermagneten (4') eine Anziehungskraft ist und der erste Rotormagnet (4) und der erste Kammermagnet (4') ein erstes Magnetlager (81) bilden, das ausgebildet ist, den Rotor (3) in einer Kipprichtung (23), die eine Drehachse in der radialen Richtung (22) aufweist, auszurichten.

5. Pumpe (1) nach einem der Ansprüche 1 bis 3, wobei die Kraft zwischen dem ersten Rotormagneten (4) und dem ersten Kammermagneten (4') eine Abstoßungskraft in einer radialen Richtung (22) ist, die senkrecht zu der axialen Richtung (21) definiert ist, und der erste Rotormagnet (4) und der erste Kammermagnet (4') ein erstes Magnetlager (81) bilden, das ausgebildet ist, den Rotor (3) in einer von der Wandung (2') der Pumpenkammer (2) in der radialen Richtung (22) beabstandeten Position zu halten.

6. Pumpe (1) nach Anspruch 5, wobei die Steuerspule (6) ein zwischen der Wicklung und der Fluidverbindung (12) und/oder auf einer dem Rotor (3) abgewandten Seite der Steuerspule (6) angeordnetes magnetisch leitfähiges Material (7) aufweist.

7. Pumpe (1) nach einem der Ansprüche 5 bis 6, umfassend einen an der Steuerspule (6) angeordneten Vorspannmagneten (6'), der ausgebildet ist, das Magnetfeld der Steuerspule (6) vorzuspannen.

8. Pumpe (1) nach einem der Ansprüche 5 bis 7, wobei der zweite Rotormagnet (5) und der zweite Kammermagnet (5') so zueinander angeordnet sind, dass eine axiale Kraft in der axialen Richtung (21) vom Einlass (10) weg auf den Rotor (3) wirkt und ein Quotient aus einer radialen Steifigkeit des zweiten Magnetlagers (82) zu einer radialen Steifigkeit des ersten Magnetlagers (81) bei Rotorverschiebung in der axialen Richtung (21), der radialen Richtung (22) oder einer Kipprichtung (23), die eine Drehachse in der radialen Richtung (22) aufweist, im Wesentlichen konstant bleibt, wobei sich die radiale Steifigkeit des ersten Magnetlagers (81) aus der Steifigkeit von Steuerspule (6) und erstem Kammermagnet (4') mit dem ersten Rotormagneten (4) ergibt.

9. Pumpe (1) nach einem der Ansprüche 4 bis 8, wobei ein Verhältnis zwischen einer radialen Steifigkeit des zweiten Magnetlagers (82) zu einer radialen Steifigkeit des ersten Magnetlagers (81) zwischen 4:1 und 1,5:1 und bevorzugt zwischen 3:1 und 1,5:1 liegt.

10. Pumpe (1) nach einem der Ansprüche 1 bis 9, wobei der Motormagnet (11) auf einer dem Einlass (10) abgewandten Seite des Impellers (40) oder auf einer dem Einlass (10) zugewandten Seite des Impellers (40) angeordnet ist.

11. Pumpe (1) nach einem der Ansprüche 1 bis 10, wobei die Steuereinheit (60) ausgebildet ist, einen Motorspulenstrom so bereitzustellen, dass das Motormagnetfeld zusammen mit dem Magnetfeld des Motormagneten (11) eine Kraft in der axialen Richtung (21) zwischen dem Motormagneten (11) und dem Motorstator (11') bewirkt.

12. Verfahren zum Betreiben einer Pumpe (1) nach einem der Ansprüche 1 bis 11, umfassend
- Bereitstellen eines Motormagnetfeldes zum Antreiben eines mit einem Rotor (3) verbundenen Impellers (40) zum Treiben eines Fluidflusses (13) von einem Einlass (10) zu einem Auslass (9) einer Pumpenkammer (2);
- Bereitstellen, mit einer Steuerspule (6) und einem am Rotor (3) angeordneten ersten Rotormagneten (4), einer steuerbaren Axialkraft auf den Rotor (3) in Richtung einer Rotationsachse (3') des Rotors (3), die eine axiale Richtung (21) definiert;
- Bereitstellen eines Steuerstroms für die Steuerspule (6), um den Rotor (3) in einer von einer Wandung (2') der Pumpenkammer (2) axial beabstandeten Position zu halten, vorzugsweise in einer zeitlich veränderlichen Position, bei der sich die Summe aller Kräfte auf den Rotor (3) außer der steuerbaren Axialkraft in der axialen Richtung (21) zu null addieren;
- Bereitstellen einer Kraft zwischen dem ersten Rotormagneten (4) und einem an der Pumpenkammer (2) nahe einer Wicklung der Steuerspule (6) angeordneten ersten Kammermagneten (4').

13. Verfahren zum Betreiben einer Pumpe (1) nach Anspruch 12, umfassend einen oder mehrere der Schritte
- Führen des Fluidflusses (13) durch eine Ausnehmung (8) im Rotor (3) um die Rotationsachse (3') des Rotors (3) vom Einlass (10) weg in Richtung Auslass (9);
- Führen des Fluidflusses (13), so dass der Fluidfluss (13) am Einlass (10) im Wesentlichen in der axialen Richtung (21) geführt wird und am Auslass (9) in einer Richtung geführt wird, die im Wesentlichen keine axiale Komponente aufweist.
- Führen eines sekundären Fluidflusses (13') zwischen dem ersten Rotormagneten (4) und der Wandung (2') der Pumpenkammer (2) und/oder Führen eines tertiären Fluidflusses (13") zwischen dem Motormagneten (11) und der Wandung (2') der Pumpenkammer (2).

14. Verfahren zum Betreiben einer Pumpe (1) nach einem der Ansprüche 12 bis 13, umfassend Erzeugen einer Abstoßungskraft zwischen einem zweiten Rotormagneten (5) und einem an der Pumpenkammer (2), dort, wo die Rotationsachse (3') auf eine Wandung (2') der Pumpenkammer (2) trifft, angeordneten zweiten Kammermagneten (5').

15. Verwendung der Pumpe (1) nach einem der Ansprüche 1 bis 11 in einem Herzunterstützungssystem.
